# EUROPEAN PATENT APPLICATION

(11) **EP 4 209 511 A1**
(43) Date of publication of application: **12.07.2023**
(21) Application number: 22382003.6
(22) Date of filing: 05.01.2022
(51) Int. Cl.: C07K 16/28, C07K 14/725, A61K 39/00

(54) **ANTI-CD84 ANTIBODIES AMD CHIMERIC ANTIGEN RECEPTORS**

(71) Applicant: Gyala Therapeutics Sociedad Limitada, 08007 Barcelona (ES); Institut d'Investigacions Biomèdiques August Pi i Sunyer (IDIBAPS), 08036 Barcelona (ES); Fundació Clinic per a la Recerca Biomèdica (FCRB), 08036 Barcelona (ES); Hospital Clínic De Barcelona (HCB), 08036 Barcelona (ES)
(72) Inventor: KLEIN GONZÁLEZ, Nela, Barcelona (ES); JUAN OTERO, Manuel, Cornellà del Llobregat (ES); VILELLA PUIG, Ramon, Barcelona (ES); PÉREZ AMILL, Lorena, Sant Quirze del Vallès (ES); RIBEIRO DOS SANTOS, Claudio Joao, Salamanca (ES)
(74) Representative: Curell Suñol S.L.P.

(57) **Abstract**

An antigen-binding domain, antibody or chimeric antigen receptor that binds to CD84.

## Description

### FIELD OF THE INVENTION

The present invention relates to anti-CD84 antibodies and chimeric antigen receptors (CARs), and their use in therapy, in particular cancer therapy. The invention also relates to methods for determining CD84 levels in a sample and for identifying a subject suitable for treatment with an anti-CD84 antibody or CAR.

### BACKGROUND TO THE INVENTION

The human CD84 protein is a "cluster of differentiation" first identified in 1993. Sequencing of CD84 has identified it as a member of the Slam *(Signaling Lymphocyte Activation Molecule)* family.

The extracellular part of the CD84 receptor contains a non-canonical IgV distal domain and an IgC2g proximal domain, a structure common to all members of the SLAM family and similar to CD2. CD84 functions as an homophilic adhesion molecule; the receptor:ligand interaction involves the IgV domain and is independent of the cytoplastic domain and there are specific differences in the homophilic interfaces that prevent the binding of CD84 to other molecules of the SLAM family.

Human CD84 is expressed in various types of immune cells. Expression levels are heterogeneous depending on the cell type and its state of differentiation or activation. CD84 appears very early during haematopoietic differentiation in bone marrow progenitor cells and is expressed in most T and B cells, but with higher expression in memory T and B lymphocytes, follicular *helper* T cells and germinal centre B cells. Myeloid antigen-presenting cells, such as monocytes and monocyte-derived dendritic cells (DCs) are also positive for CD84. Granulocytes also express significant levels of CD84 with the highest expression on basophils and mast cells. Among all haematopoietic cells, platelets express the highest levels of CD84, which was initially proposed as an aggregation-induced signalling receptor implicated in stabilizing platelet-platelet interactions. However, studies with CD84-deficient mice indicate that it does not play a significant role in haemostatic and thrombotic function. CD84 expression is low in natural killer cells (NK) and absent in red blood cells.

CD84 signalling can activate or inhibit leukocyte function depending on the cell type and its stage of activation or differentiation. CD84-mediated signalling regulates diverse immunological processes, including T-cell cytokine secretion, natural killer-cell cytotoxicity, monocyte activation, autophagy, cognate T:B interactions and B-cell tolerance at the level of germinal centres.

Alterations in CD84 have been related to autoimmune disorders. For example, specific allelic variations in CD84 are associated with autoimmune diseases such as systemic lupus erythematosus and rheumatoid arthritis (RA). Typical treatment for such autoimmune diseases often involves disease-modifying anti-rheumatic drugs (DMARDS), which are not curative but stop or slow down symptoms and/or disease progression.

CD84 is expressed in certain cancers, in particular haematological malignancies, such as chronic lymphocytic leukaemia (CLL).

Haematological malignancies are often treated by chemotherapy or radiotherapy, which have known adverse side effects. Accordingly, there remains a significant need for improved treatments for haematological malignancies.

### SUMMARY OF THE INVENTION

The inventors have determined that CD84 is overexpressed in a range of cancer cell lines derived from malignant haematological diseases, including Burkitt's lymphoma, acute myeloid leukaemia (AML), chronic myeloid leukaemia (CML), B-cell acute lymphoblastic leukaemia (B-ALL), T-cell acute lymphoblastic leukaemia (T-ALL) and histiocytic lymphoma.

The inventors have studied and developed a range of antibodies and chimeric antigen receptors (CARs) that bind CD84.

In particular, the inventors have characterised the cellular expression of the CARs and functionally characterised CAR-T cells, for example through determination of their proliferation, cytokine production and their cytotoxicity against a range of target cancer cell lines. Moreover, the inventors have demonstrated that chimeric antigen receptors of the present invention can induce cytotoxicity in cancer cells expressing CD84.

Accordingly, the present invention provides an antigen-binding domain comprising:
a) a heavy chain variable region (VH) having complementarity determining regions (CDRs) with the sequences: CDR1 - NYWIN (SEQ ID NO: 1); CDR2 - DIYPVSGTTNYNEKFKR (SEQ ID NO: 2); and CDR3 - GTGRFAY (SEQ ID NO: 3); or variants thereof each having up to three amino acid substitutions, additions or deletions; and
   a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6); or variants thereof each having up to three amino acid substitutions, additions or deletions;
b) a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9), or variants thereof each having up to three amino acid substitutions, additions or deletions; and
   a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12), or variants thereof each having up to three amino acid substitutions, additions or deletions;
c) a heavy chain variable region (VH) having complementarity determining regions (CDRs) with the sequences: CDR1 -GFTFSSYA (SEQ ID NO: 13); CDR2 - ISGSGGST (SEQ ID NO: 14); and CDR3-AKWDCSDGRCYWAY (SEQ ID NO: 15), or variants thereof each having up to three amino acid substitutions, additions or deletions; and
   a light chain variable region (VL) having CDRs with the sequences: CDR1 - NIESKD (SEQ ID NO: 16); CDR2 - DDA (SEQ ID NO: 17); and CDR3 - QVWDSSSDHVV (SEQ ID NO: 18), or variants thereof each having up to three amino acid substitutions, additions or deletions;
d) a heavy chain variable region (VH) having complementarity determining regions (CDRs) with the sequences: CDR1 -GFTFSSYP (SEQ ID NO: 19); CDR2 - ISYHGRNK (SEQ ID NO: 20); and CDR3 - ARDRDATPGGTGVGNHGMAV (SEQ ID NO: 21), or variants thereof each having up to three amino acid substitutions, additions or deletions; and
   a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24), or variants thereof each having up to three amino acid substitutions, additions or deletions;
e) a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3-AKWDCSDGRCYWAY (SEQ ID NO: 27), or variants thereof each having up to three amino acid substitutions, additions or deletions; and
   a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30), or variants thereof each having up to three amino acid substitutions, additions or deletions;
f) a heavy chain variable region (VH) having complementarity determining regions (CDRs) with the sequences: CDR1 -TSGMGVG (SEQ ID NO: 31); CDR2 - HIWWDDVKRYNPALKS (SEQ ID NO: 32); and CDR3 - MRTSYYFDY (SEQ ID NO: 33), or variants thereof each having up to three amino acid substitutions, additions or deletions; and
   a light chain variable region (VL) having CDRs with the sequences: CDR1 - RASENIFSSLA (SEQ ID NO: 34); CDR2 - NAKTLAE (SEQ ID NO: 35); and CDR3 - QHHYATPFT (SEQ ID NO: 36), or variants thereof each having up to three amino acid substitutions, additions or deletions;
g) a heavy chain variable region (VH) having complementarity determining regions (CDRs) with the sequences: CDR1 - SYWIN (SEQ ID NO: 37); CDR2 - DIYLGSGSTNYNEKFKS (SEQ ID NO: 38); and CDR3 - SGGYLGY (SEQ ID NO: 39), or variants thereof each having up to three amino acid substitutions, additions or deletions; and
   a light chain variable region (VL) having CDRs with the sequences: CDR1 - RASQSVSTSSYSYMH (SEQ ID NO: 40); CDR2 - FASNLES (SEQ ID NO: 41); and CDR3 - QHSWEIPYT (SEQ ID NO: 42), or variants thereof each having up to three amino acid substitutions, additions or deletions;
h) a heavy chain variable region (VH) having complementarity determining regions (CDRs) with the sequences: CDR1 - NYWIG (SEQ ID NO: 43); CDR2 - DIYPGGGYTNYNENFKG (SEQ ID NO: 44); and CDR3 - STTYYSSYWCFDV (SEQ ID NO: 45), or variants thereof each having up to three amino acid substitutions, additions or deletions; and
   a light chain variable region (VL) having CDRs with the sequences: CDR1 - KSSQSLLNSGNQANYLA (SEQ ID NO: 46); CDR2 - GASTRES (SEQ ID NO: 47); and CDR3 - QNDHSYPFT (SEQ ID NO: 48), or variants thereof each having up to three amino acid substitutions, additions or deletions;
i) a heavy chain variable region (VH) having complementarity determining regions (CDRs) with the sequences: CDR1 - RYWMS (SEQ ID NO: 49); CDR2 - EINPDSSTINYTPSLKD (SEQ ID NO: 50); and CDR3 - PGPTVVATYWYFDV (SEQ ID NO: 51), or variants thereof each having up to three amino acid substitutions, additions or deletions; and
   a light chain variable region (VL) having CDRs with the sequences: CDR1 - RSSQSIVHSNGNTYLE (SEQ ID NO: 52); CDR2 - KVSSRFS (SEQ ID NO: 53); and CDR3 - FQGSHVPRT (SEQ ID NO: 54), or variants thereof each having up to three amino acid substitutions, additions or deletions;
j) a heavy chain variable region (VH) having complementarity determining regions (CDRs) with the sequences: CDR1 - RYWIN (SEQ ID NO: 55); CDR2 - DIYPGSGSTNYNEKFKS (SEQ ID NO: 56); and CDR3 - DTTIAY (SEQ ID NO: 57), or variants thereof each having up to three amino acid substitutions, additions or deletions; and
   a light chain variable region (VL) having CDRs with the sequences: CDR1 - RASQSVTTSRYSYMH (SEQ ID NO: 58); CDR2 - FASNLES (SEQ ID NO: 59); and CDR3 - QHSWEIPYT (SEQ ID NO: 60), or variants thereof each having up to three amino acid substitutions, additions or deletions;
k) a heavy chain variable region (VH) having complementarity determining regions (CDRs) with the sequences: CDR1 - GYNMN (SEQ ID NO: 131); CDR2 - NIDPYYGGTNYNQKFKG (SEQ ID NO: 132); and CDR3 - GLLSGSFPY (SEQ ID NO: 133), or variants thereof each having up to three amino acid substitutions, additions or deletions; and
   a light chain variable region (VL) having CDRs with the sequences: CDR1 - RASENIYSYLA (SEQ ID NO: 134); CDR2- NAKTLAE (SEQ ID NO: 135); and CDR3 - QHHYGSPLT (SEQ ID NO: 136), or variants thereof each having up to three amino acid substitutions, additions or deletions;
l) a heavy chain variable region (VH) having complementarity determining regions (CDRs) with the sequences: CDR1 - RSWMS (SEQ ID NO: 137); CDR2 - EINPDSSTINYTPSLKD (SEQ ID NO: 138); and CDR3 - FYDGYSIYWYFDV (SEQ ID NO: 139), or variants thereof each having up to three amino acid substitutions, additions or deletions; and
   a light chain variable region (VL) having CDRs with the sequences: CDR1 - RSSQSIVHSNGDTYLE (SEQ ID NO: 140); CDR2 - KVSNRFS (SEQ ID NO: 141); and CDR3 - FQGSHVPRT (SEQ ID NO: 142), or variants thereof each having up to three amino acid substitutions, additions or deletions;
m) a heavy chain variable region (VH) having complementarity determining regions (CDRs) with the sequences: CDR1 - TSGMGVG (SEQ ID NO: 143); CDR2 - HIWWDDVKRYNPALRS (SEQ ID NO: 144); and CDR3 - IAVTYFFDF (SEQ ID NO: 145), or variants thereof each having up to three amino acid substitutions, additions or deletions; and
   a light chain variable region (VL) having CDRs with the sequences: CDR1 - RASENIFSSFA (SEQ ID NO: 146); CDR2 - NARTLAE (SEQ ID NO: 147); and CDR3 - QHHYASPFT (SEQ ID NO: 148), or variants thereof each having up to three amino acid substitutions, additions or deletions;
n) a heavy chain variable region (VH) having complementarity determining regions (CDRs) with the sequences: CDR1 - TSGMGVG (SEQ ID NO: 149); CDR2 - HIWWDDVKRYNPALKS (SEQ ID NO: 150); and CDR3 - MSTSYYFDY (SEQ ID NO: 151), or variants thereof each having up to three amino acid substitutions, additions or deletions; and
   a light chain variable region (VL) having CDRs with the sequences: CDR1 - KASQSLFTSVA (SEQ ID NO: 152); CDR2 - SASYRYT (SEQ ID NO: 153); and CDR3 - QQHYSSPFT (SEQ ID NO: 154), or variants thereof each having up to three amino acid substitutions, additions or deletions; or
o) a heavy chain variable region (VH) having complementarity determining regions (CDRs) with the sequences: CDR1 - IYAMN (SEQ ID NO: 155); CDR2 - RIRSKSNNYARFYADSVKD (SEQ ID NO: 156); and CDR3 - PLRSYFSMDY (SEQ ID NO: 157), or variants thereof each having up to three amino acid substitutions, additions or deletions; and
   a light chain variable region (VL) having CDRs with the sequences: CDR1 - KASENVDTYVS (SEQ ID NO: 158); CDR2 - GASNRYT (SEQ ID NO: 159); and CDR3 - GQTYSYPWT (SEQ ID NO: 160), or variants thereof each having up to three amino acid substitutions, additions or deletions.

In some embodiments, the antigen-binding domain comprises:
a) a VH domain having the sequence of SEQ ID NO: 61; and a VL domain having the sequence of SEQ ID NO: 62 or 108;
b) a VH domain having the sequence of SEQ ID NO: 63; and a VL domain having the sequence of SEQ ID NO: 64 or 109;
c) a VH domain having the sequence of SEQ ID NO: 65; and a VL domain having the sequence of SEQ ID NO: 66;
d) a VH domain having the sequence of SEQ ID NO: 68; and a VL domain having the sequence of SEQ ID NO: 69;
e) a VH domain having the sequence of SEQ ID NO: 71; and a VL domain having the sequence of SEQ ID NO: 72;
f) a VH domain having the sequence of SEQ ID NO: 74; and a VL domain having the sequence of SEQ ID NO: 75;
g) a VH domain having the sequence of SEQ ID NO: 76; and a VL domain having the sequence of SEQ ID NO: 77;
h) a VH domain having the sequence of SEQ ID NO: 78; and a VL domain having the sequence of SEQ ID NO: 79;
i) a VH domain having the sequence of SEQ ID NO: 80; and a VL domain having the sequence of SEQ ID NO: 81;
j) a VH domain having the sequence of SEQ ID NO: 82; and a VL domain having the sequence of SEQ ID NO: 83;
k) a VH domain having the sequence of SEQ ID NO: 161; and a VL domain having the sequence of SEQ ID NO: 162;
l) a VH domain having the sequence of SEQ ID NO: 163; and a VL domain having the sequence of SEQ ID NO: 164;
m) a VH domain having the sequence of SEQ ID NO: 165; and a VL domain having the sequence of SEQ ID NO: 166;
n) a VH domain having the sequence of SEQ ID NO: 167; and a VL domain having the sequence of SEQ ID NO: 168; or
o) a VH domain having the sequence of SEQ ID NO: 169; and a VL domain having the sequence of SEQ ID NO: 170;
or variants thereof each having at least 75%, at least 80%, at least 85%, at least 90%, at least 95%, at least 96%, at least 97%, at least 98% or at least 99%, preferably at least 90%, sequence identity thereto.

Suitably, the antigen-binding domain is a CD84-binding domain.

In some embodiments, the antigen-binding domain is an scFv.

In another aspect, the invention provides an antibody comprising the antigen-binding domain of the invention.

In another aspect, the invention provides a chimeric antigen receptor (CAR) comprising the antigen-binding domain of the invention.

Suitably, the antigen-binding domain, antibody or CAR of the invention binds to CD84.

In some embodiments, the CAR is an scFv CAR.

In some embodiments, the CAR comprises a CD8a or a CD28 transmembrane domain. In preferred embodiments, the CAR comprises a CD8a transmembrane domain.

The antigen-binding domain and the transmembrane domain may be connected by a spacer. In some embodiments, the spacer comprises a CD8a hinge or a CD28 hinge. In preferred embodiments, the CAR comprises a CD8a hinge.

The CAR may comprise one or more, for example two or three, intracellular signalling domains. In preferred embodiments, the CAR comprises a CD3-zeta signalling domain.

The CAR may comprise one or more, for example two or three, co-stimulatory domains. In some embodiments, the CAR comprises one or more co-stimulatory domains selected from the group consisting of a 4-1BB co-stimulatory domain, a CD28 co-stimulatory domain, a OX40 co-stimulatory domain and an ICOS co-stimulatory domain.

In preferred embodiments, the CAR comprises a 4-1BB co-stimulatory domain. In some embodiments, the CAR comprises a CD28 co-stimulatory domain. In some embodiments, the CAR comprises an OX40 co-stimulatory domain. In some embodiments, the CAR comprises an ICOS co-stimulatory domain.

In preferred embodiments, the CAR comprises a 4-1BB co-stimulatory domain and a CD3-zeta signalling domain.

In preferred embodiments, the CAR comprises the antigen-binding domain of the invention, a CD8a transmembrane domain, a 4-1BB co-stimulatory domain and a CD3-zeta signalling domain.

In another aspect, the invention provides a polynucleotide comprising one or more nucleotide sequences encoding the antigen-binding domain, the antibody or the CAR of the invention.

In another aspect, the invention provides a vector comprising the polynucleotide of the invention.

In some embodiments, the vector is a viral vector. In some embodiments, the vector is a retroviral vector or lentiviral vector, preferably a lentiviral vector.

In another aspect, the invention provides a cell comprising the polynucleotide or the vector of the invention.

In another aspect, the invention provides a cell comprising the antigen-binding domain or the CAR of the invention.

The cell may comprise a further (second) CAR.

In another aspect, the invention provides a cell comprising a first CAR and a second CAR, wherein the first CAR is the CAR of the invention.

In another aspect, the invention provides a composition comprising a first cell and a second cell, wherein the first cell comprises a first CAR and the second cell comprises a second CAR, and wherein the first CAR is the CAR of the invention.

In some embodiments, the first CAR and the second CAR are different. In some embodiments, the first CAR and the second CAR bind to different antigens.

In another aspect, the invention provides a cell comprising a tandem CAR, wherein the tandem CAR comprises a first antigen-binding domain (e.g. scFv) and a second antigen-binding domain (e.g. scFv), and wherein the first antigen-binding domain (e.g. scFv) is the antigen-binding domain (e.g. scFv) of the invention.

In some embodiments, the first antigen-binding domain (e.g. scFv) and the second antigen-binding domain (e.g. scFv) are different. In some embodiments, the first antigen-binding domain (e.g. scFv) and the second antigen-binding domain (e.g. scFv) bind to different antigens.

In some embodiments, the second CAR is an anti-CD19 CAR, an anti-CD20 CAR or an anti-CD22 CAR (e.g. for the treatment of B-cell malignancies); an anti-CD33 CAR or an anti-CD123 CAR (e.g. for the treatment of AML); or an anti-CD7 CAR (e.g. for the treatment of T-ALL).

In some embodiments, the second antigen-binding domain (e.g. scFv) is an anti-CD19 antigen-binding domain (e.g. scFv), an anti-CD20 antigen-binding domain (e.g. scFv) or an anti-CD22 antigen-binding domain (e.g. scFv) (e.g. for the treatment of B-cell malignancies); an anti-CD33 antigen-binding domain (e.g. scFv) or an anti-CD123 antigen-binding domain (e.g. scFv) (e.g. for the treatment of AML); or an anti-CD7 antigen-binding domain (e.g. scFv) (e.g. for the treatment of T-ALL).

In some embodiments, the cell is a T cell or NK cell, preferably a T cell. In some embodiments, the T cell is an autologous or allogeneic T cell. In some embodiments, the NK cell is an autologous or allogeneic NK cell.

NK cells may, for example, be sourced from any suitable tissue sample, cell line or stem cell source. In some embodiments, the NK cell (preferably allogeneic NK cell) is from cord blood (CB), induced pluripotent stem cells (iPSCs), bone marrow (BM), human embryonic stem cells (hESCs), a cell line (e.g. NK92 or YT), or peripheral blood (PB) (e.g. autologous or allogeneic). Preferably, the NK cell is from CB.

In another aspect, the invention provides a pharmaceutical composition comprising the antigen-binding domain, the antibody, the CAR, the polynucleotide, the vector or the cell of the invention.

In another aspect, the invention provides the antigen-binding domain, the antibody, the CAR, the polynucleotide, the vector, the cell or the pharmaceutical composition of the invention for use in therapy.

In some embodiments, the therapy is treatment of cancer.

In another aspect, the invention provides a method for treating a disease comprising administering the antigen-binding domain, the antibody, the CAR, the polynucleotide, the vector, the cell or the pharmaceutical composition of the invention to a subject in need thereof.

In some embodiments, the disease is cancer.

In another aspect, the invention provides use of the antigen-binding domain, the antibody, the CAR, the polynucleotide, the vector, the cell or the pharmaceutical composition of the invention for the manufacture of a medicament.

In some embodiments, the medicament is for treating a disease, preferably cancer.

In preferred embodiments, the cancer is a haematological malignancy.

In preferred embodiments, the cancer cells express CD84, for example the haematological malignancy may be a CD84-expressing haematological malignancy.

In some embodiments, the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

In some embodiments, the cancer is selected from the group consisting of chronic lymphocytic leukaemia (CLL), diffuse large B-cell lymphoma (DLBCL), Burkitt's lymphoma, follicular lymphoma, mantle cell lymphoma, B-cell acute lymphoblastic leukaemia (B-ALL), T-cell acute lymphoblastic leukaemia/lymphoma (T-ALL), acute myeloid leukemia (AML) and histiocytic sarcoma.

In preferred embodiments, the cancer is selected from the group consisting of CLL, B-cell lymphoma, B-ALL, T-ALL and AML.

In another aspect, the invention provides use of the antigen-binding domain or the antibody of the invention for determining CD84 expression level in a sample, optionally for analysing a CD84 expression level in a sample from a subject.

In another aspect, the invention provides a method for identifying a subject suitable for treatment with an anti-CD84 CAR or antibody, wherein the method comprises determining a CD84 expression level in a sample isolated from the subject, wherein the CD84 expression level is determined using the antigen-binding domain or the antibody of the invention.

In some embodiments, the sample is a blood sample.

### DESCRIPTION OF THE DRAWINGS

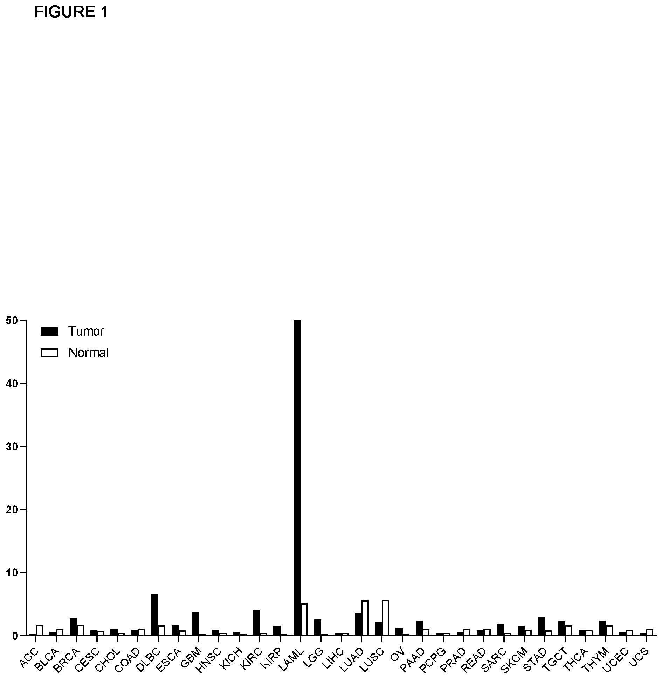

**Figure 1****.** Bar plot representing CD84 (alias LY9B, SLAMF5, hCD84, mCD84) gene expression profile across all tumour samples and paired normal tissues. The height of bar represents the median expression of certain tumour type or normal tissue. From http://gepia.cancer-pku.cn/detail.php?gene=CD84.

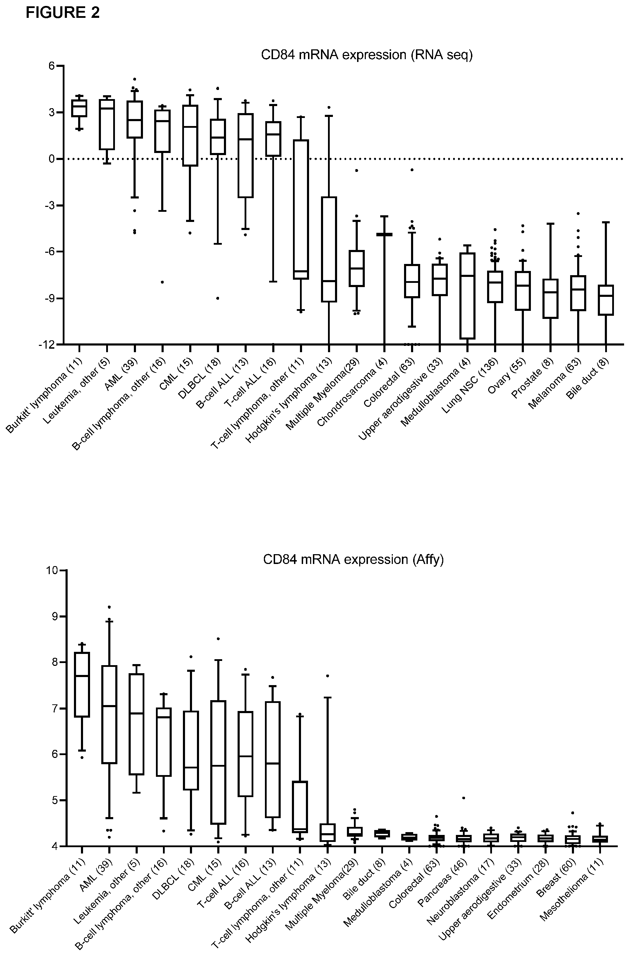

**Figure 2****.** Box and whisker (10-90 percentile) representation of CD84 mRNA expression measured by RNA sequencing (top) or Affymetrix microarrays (bottom) in cell lines obtained from different tumour types. From https://portals.broadinstitute.org/ccle. The figure only shows the 20 tumour types with the highest CD84 expression.

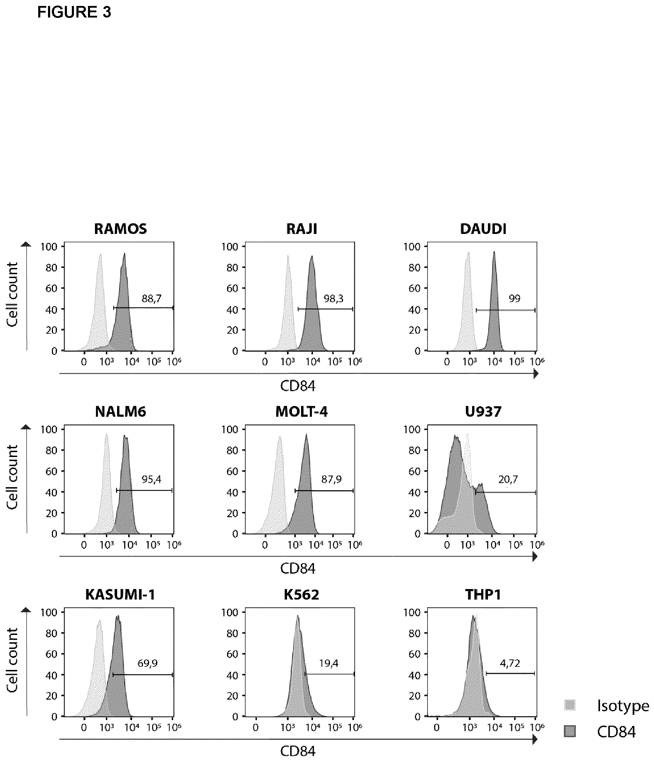

**Figure 3****.** Expression of CD84 in different cell lines. The histogram in light grey represents the staining with an isotype-matched control antibody and the histogram in dark grey the staining with the specific CD84 antibody.

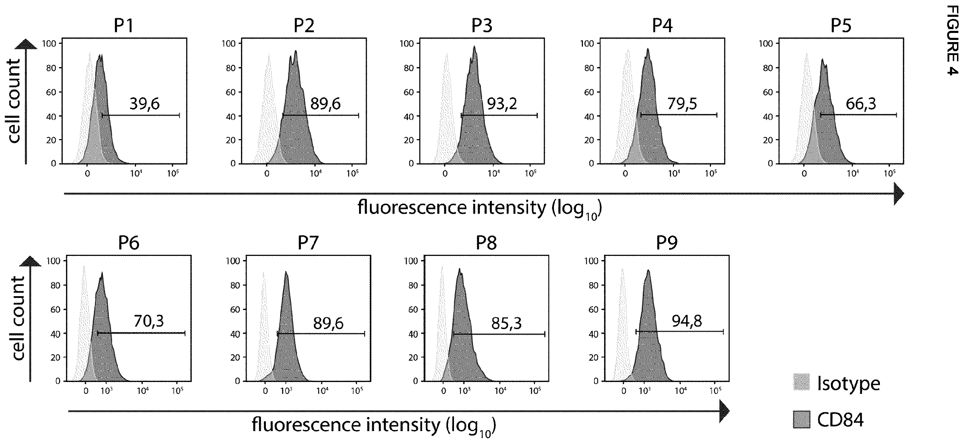

**Figure 4****.** Expression of CD84 in nine samples from patients diagnosed with chronic lymphocytic leukaemia assessed by flow cytometry. The histogram in light grey represents the staining with an isotype-matched control antibody and the histogram in dark grey the staining with the specific CD84 antibody.

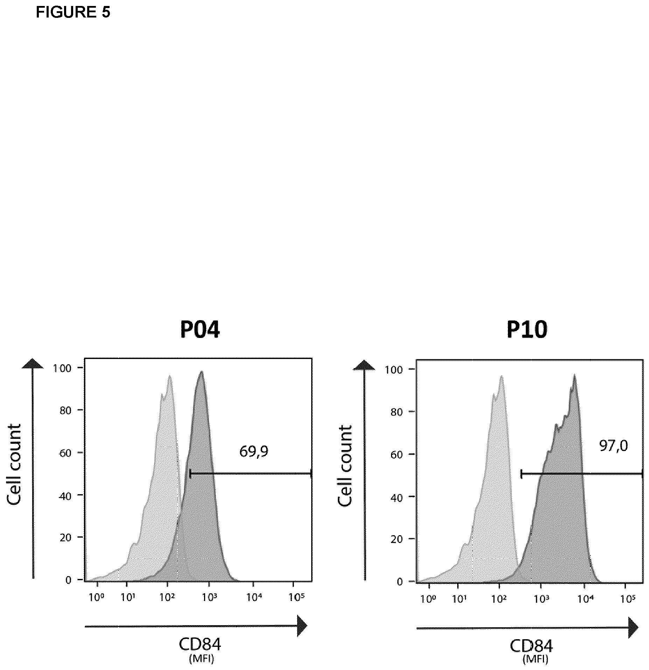

**Figure 5****.** Expression of CD84 in ten samples from patients diagnosed with acute myeloid leukaemia was assessed by flow cytometry. One representative example is shown for moderate expression of CD84, patient P04, and one for high expression of CD84, patient P10. The histogram in light grey represents the staining with an isotype-matched control antibody and the histogram in dark grey the staining with the specific CD84 antibody.

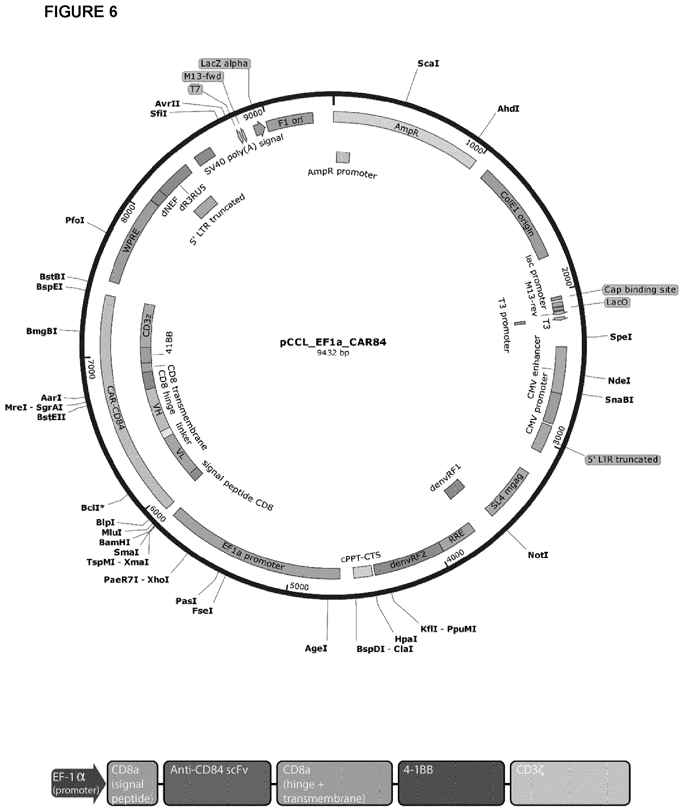

**Figure 6****.** Map of the pCCL-EF1α_CAR84 plasmid vector showing where the CD84 CAR sequence has been inserted (top). Scheme of the anti-CD84 2^{nd} generation CAR construct (bottom).

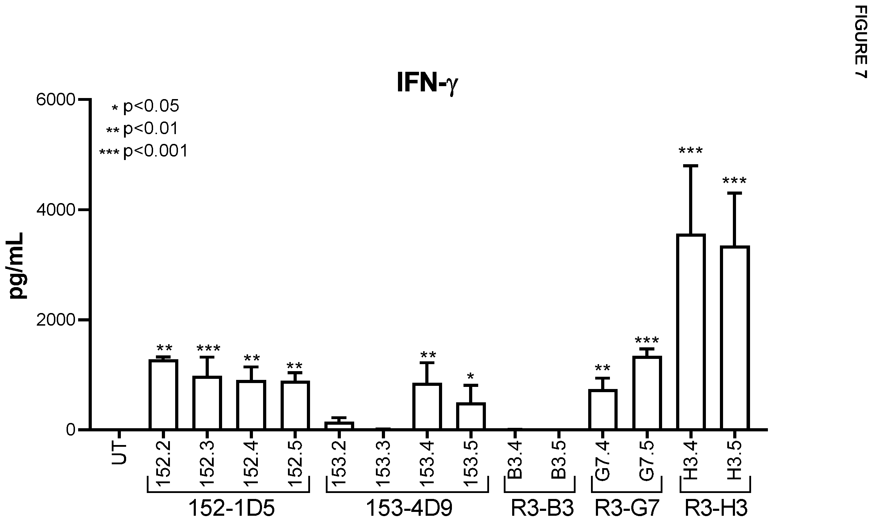

**Figure 7****.** IFN-y secretion by CD84-targeting CAR-T cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

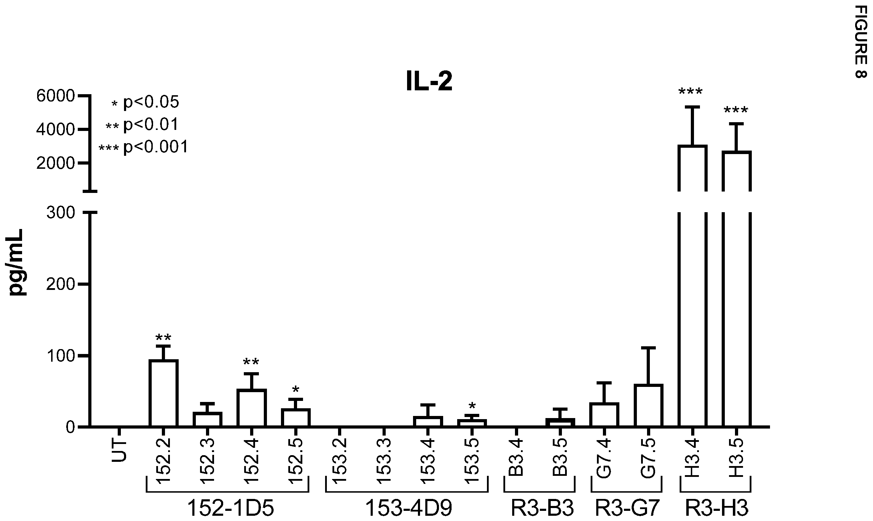

**Figure 8****.** IL-2 secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

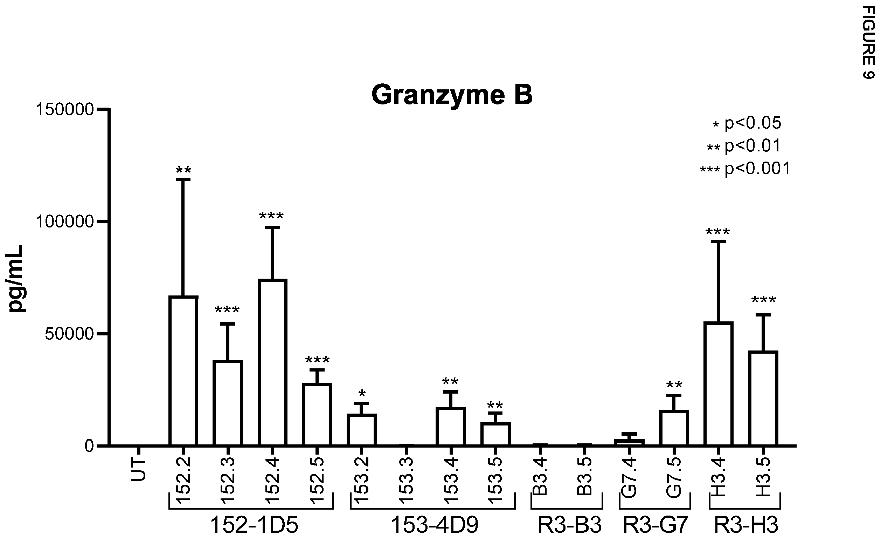

**Figure 9****.** Granzyme B secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

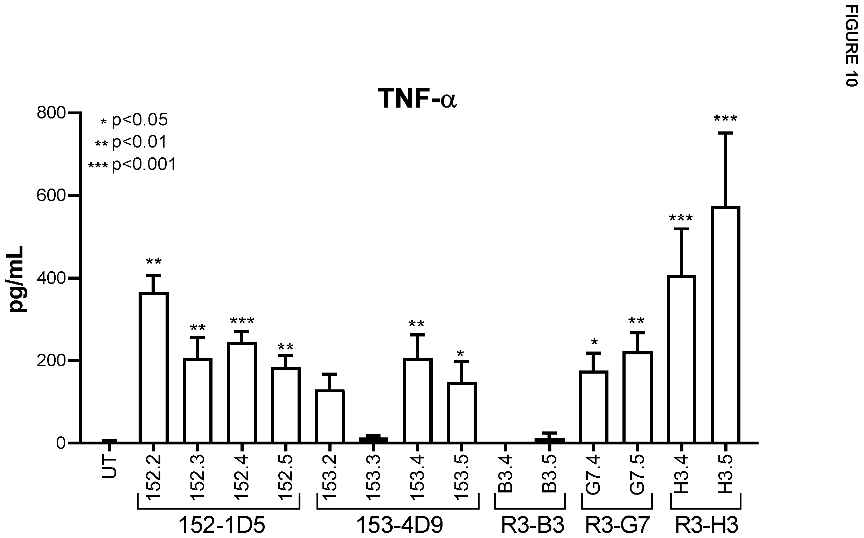

**Figure 10****.** TNF-α secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

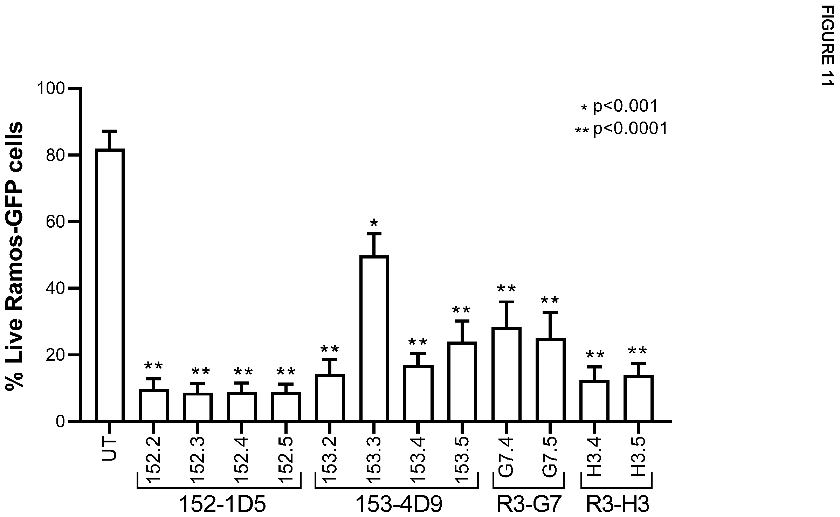

**Figure 11****.** Cytotoxicity assay of different CD84-targeting CART cells vs. Ramos-GFP⁺ cells after an incubation period of 24 hours at an effector:target ratio of 2:1. Percentage of target surviving cells relative to untreated cells (target cells alone) is shown. Mean of 8 independent experiments ± SEM. UT: untransduced T cells. Statistical significance was determined with an ordinary one-way ANOVA (multiple comparisons to UT): ** p < 0.001, * p < 0.01.

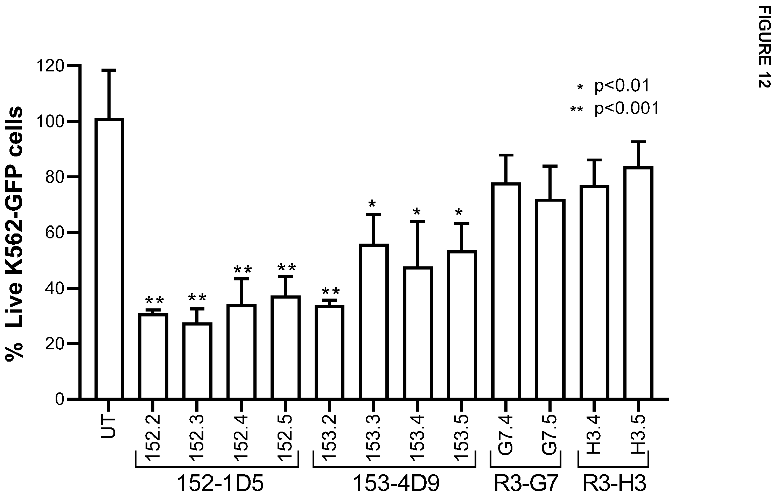

**Figure 12****.** Cytotoxicity assay of different CD84-targeting CART cells vs. K562-GFP⁺ cells after an incubation period of 24 hours at an effector:target ratio of 2:1. Percentage of target surviving cells relative to untreated cells (target cells alone) is shown. Mean of 8 independent experiments ± SEM. UT: untransduced T cells. Statistical significance was determined with an ordinary one-way ANOVA (multiple comparisons to UT): ** p < 0.001, * p < 0.01.

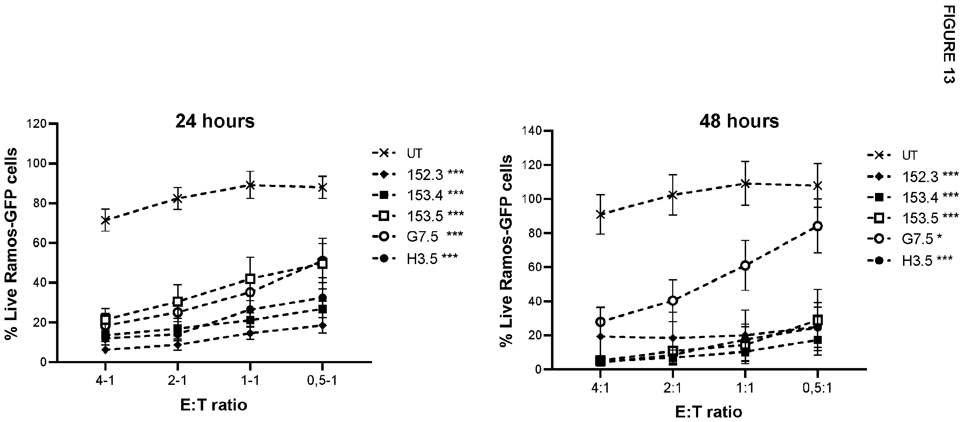

**Figure 13****.** Cytotoxicity assay of different CD84-targeting CART cells vs. Ramos-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of 5 independent experiments ± SEM. UT: untransduced T cells. Statistical significance was determined with an ordinary one-way ANOVA (multiple comparisons to UT): *** p < 0.001, * p < 0.05.

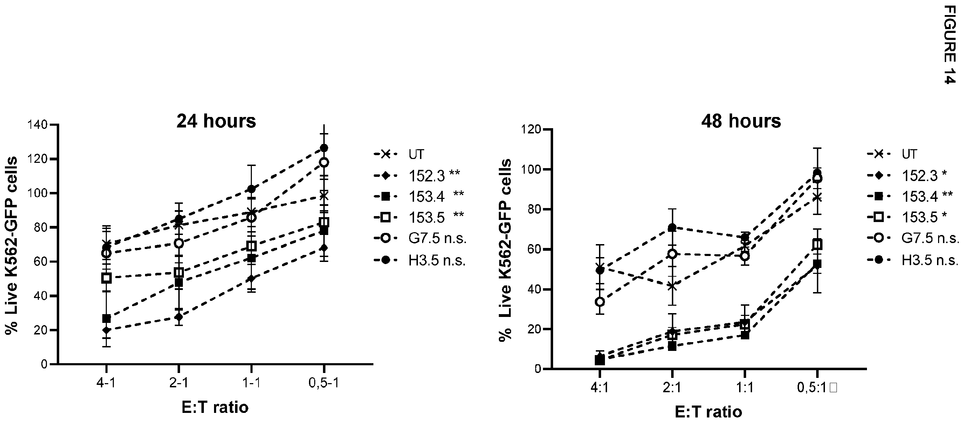

**Figure 14****.** Cytotoxicity assay of different CD84-targeting CART cells vs. K562-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. UT: untransduced T cells. Mean of 5 independent experiments ± SEM. Statistical significance was determined with an ordinary one-way ANOVA (multiple comparisons to UT): ** p <0.01, * p < 0.05, n.s.: non significant.

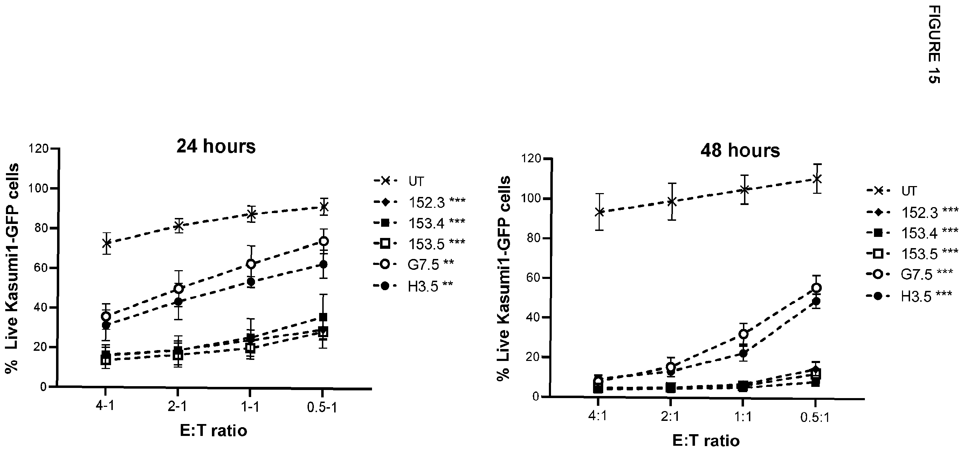

**Figure 15****.** Cytotoxicity assay of different CD84-targeting CART cells vs. Kasumi1-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of 3 independent experiments ± SEM, UT: untransduced T cells. Statistical significance was determined with an ordinary one-way ANOVA (multiple comparisons to UT): *** p < 0.001, ** p < 0.01, * p < 0.05.

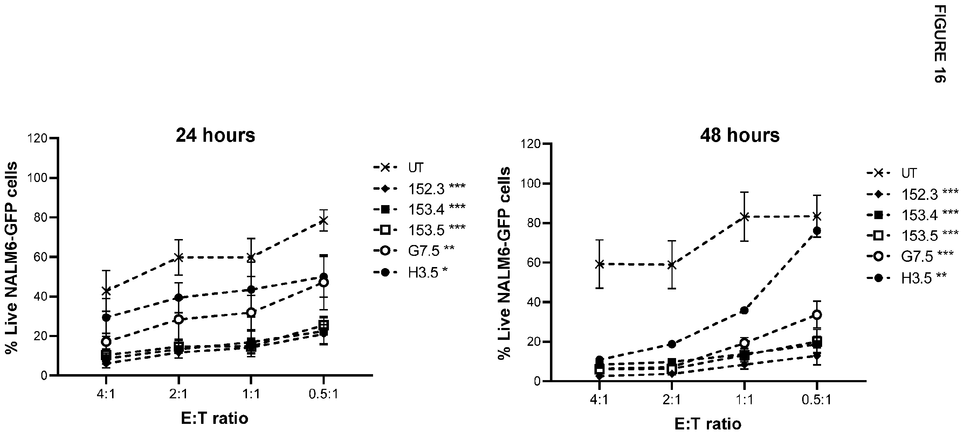

**Figure 16****.** Cytotoxicity assay of different CD84-targeting CART cells vs. NALM6-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone), is shown. Mean of 3 independent experiments ± SEM. UT: untransduced T cells. Statistical significance was determined with an ordinary one-way ANOVA (multiple comparisons to UT): *** p < 0.001, ** p < 0.01, * p < 0.05.

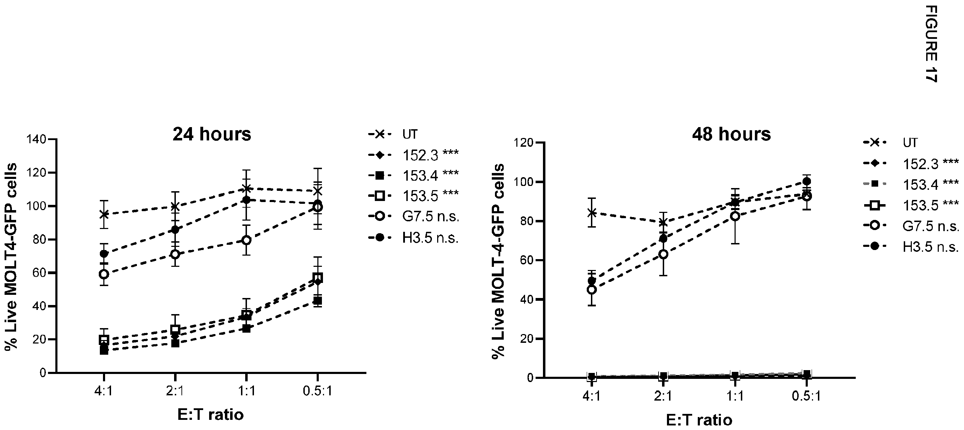

**Figure 17****.** Cytotoxicity assay of different CD84-targeting CART cells vs. MOLT4-GFP⁺ cells after an incubation period of 24 and 48 hours at effector:target ratios of 4:1, 2:1, 1:1 and 0.5:1. Percentage of target surviving cells relative to untreated cells (target cells alone), is shown. Mean of 4 independent experiments ± SEM. UT: untransduced T cells. Statistical significance was determined with an ordinary one-way ANOVA (multiple comparisons to UT): ** p < 0.01, * p < 0.05, ns: non significant.

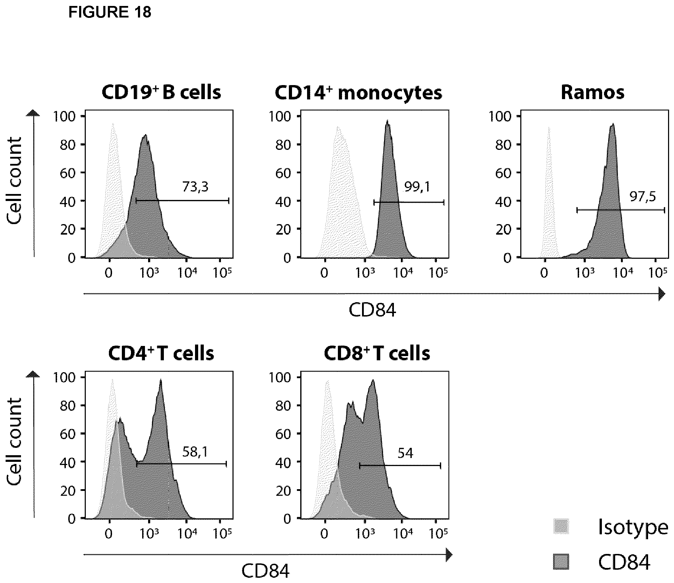

**Figure 18**. Expression of CD84 in peripheral blood mononuclear cells (PBMC) assessed by flow cytometry. The histogram in light grey represents the staining with an isotype-matched control antibody and the histogram in dark grey the staining with the specific CD84 antibody. PBMC were stained with specific antibodies for CD4, CD8, CD19, and CD14.

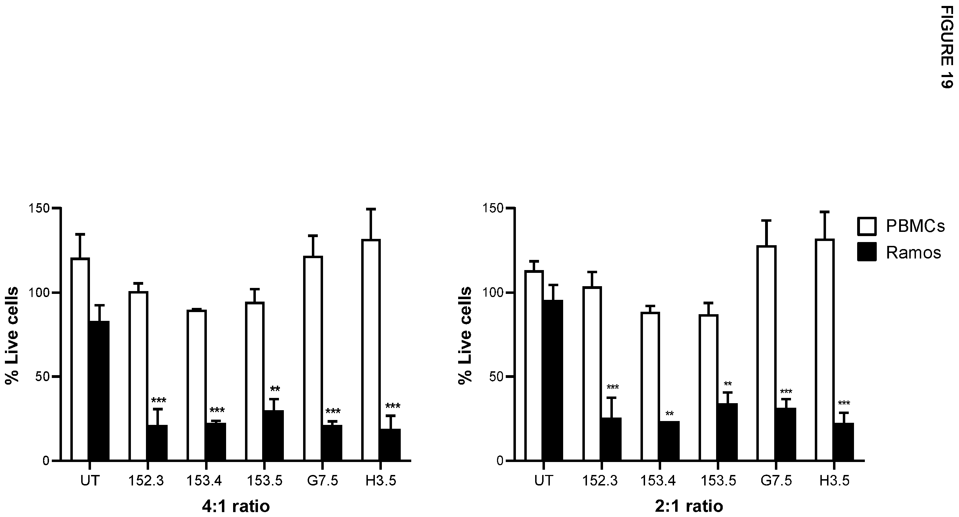

**Figure 19****.** Cytotoxicity assay of different CD84-targeting CART cells vs. PBMC labelled with CFSE⁺ and Ramos-GFP⁺ cells after an incubation period of 16 hours. Percentage of target surviving cells relative to untreated cells (target cells alone) is shown. Mean of 3 independent experiments from 3 different donors ± SEM. UT: untransduced T cells. Statistical significance was determined with a paired T test (compared to UT): ** p < 0.01, n.s. = non significant.

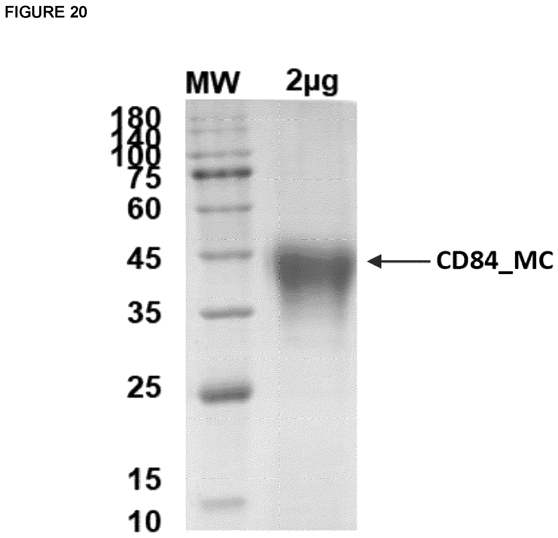

**Figure 20****.** SDS PAGE gel image of CD84 antigen. Reduced SDS PAGE with Coomassie blue staining.

### DETAILED DESCRIPTION OF THE INVENTION

The terms "comprising", "comprises" and "comprised of' as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

### CD84

CD84 (also known as LY9B and SLAMF5) is a membrane glycoprotein that is a member of the signalling lymphocyte activation molecule (SLAM) family, which itself is a subset of the larger CD2 cell-surface receptor subgroup of the Ig superfamily.

The extracellular part of the CD84 receptor contains a non-canonical IgV distal domain and an IgC2g proximal domain, a structure common to all members of the SLAM family. CD84 functions as an homophilic adhesion molecule and is expressed in numerous immune cell types. The receptor:ligand interaction involves the IgV domain and is independent of the cytoplastic domain. There are specific differences in the homophilic interfaces that prevent the binding of CD84 to other molecules of the SLAM family.

The inventors have determined that CD84 is overexpressed in a range of cell lines derived from malignant haematological diseases, including Burkitt's lymphoma, acute myeloid leukaemia (AML), chronic myeloid leukaemia (CML), B-cell acute lymphoblastic leukaemia (B-ALL), T-cell acute lymphoblastic leukaemia (T-ALL) and histiocytic lymphoma.

Studies have suggested that CD84 is overexpressed in chronic lymphocytic leukaemia (CLL).

An example amino acid sequence of CD84 is:

MAQHHLWILLLCLQTWPEAAGKDSEIFTVNGILGESVTFPVNIQEPRQVKIIAWTSKTSVAYVTPGDSE
TAPVVTVTHRNYYERIHALGPNYNLVISDLRMEDAGDYKADINTQADPYTTTKRYNLQIYRRLGKPKIT
QSLMASVNSTCNVTLTCSVEKEEKNVTYNWSPLGEEGNVLQIFQTPEDQELTYTCTAQNPVSNNSDSIS
ARQLCADIAMGFRTHHTGLLSVLAMFFLLVLILSSVFLFRLFKRRQGRIFPEGSCLNTFTKNPYAASKK
TIYTYIMASRNTQPAESRIYDEILQSKVLPSKEEPVNTVYSEVQFADKMGKASTQDSKPPGTSSYEIVI

(SEQ ID NO: 126)

### Antigen-binding domain

An antigen-binding domain may be a protein or peptide that possesses the ability to recognise and bind to an antigen. The antigen-binding domain includes any naturally occurring, synthetic, semi-synthetic or recombinantly produced binding partner for an antigen of interest. Illustrative antigen-binding domains include antibodies or antibody fragments or derivatives, extracellular domains of receptors (e.g. TCRs), ligands for cell surface molecules/receptors, or receptor binding domains thereof.

In preferred embodiments, the antigen-binding domain is, or is derived from, an antibody. An antibody-derived domain can be a fragment of an antibody or a genetically engineered product of one or more fragments of the antibody, which fragment is involved in binding with the antigen. Examples include a variable region (Fv), a complementarity determining region (CDR), a Fab, a single chain variable fragment (scFv), a heavy chain variable region (VH), a light chain variable region (VL) and a camelid antibody (VHH).

The antigen-binding domain may be non-human (e.g. murine), chimeric, humanised or fully human.

In preferred embodiments, the antigen-binding domain is a single chain variable fragment (scFv). The scFv may be, for example, a murine, human or humanised scFv.

The term "complementarity determining region" (CDR) with regard to an antibody or antigen-binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain three CDRs.

"Heavy chain variable region" (VH) refers to the variable fragment of the heavy chain of an antibody that contains three CDRs interposed between flanking stretches known as framework regions, which are more highly conserved than the CDRs and form a scaffold to support the CDRs.

"Light chain variable region" (VL) refers to the variable fragment of the light chain of an antibody that contains three CDRs interposed between framework regions.

"Fv" refers to the smallest fragment of an antibody to bear the complete antigen-binding site. An Fv consists of the variable region of a single light chain bound to the variable region of a single heavy chain.

"Single chain variable fragment" (scFv) refers to an engineered antibody consisting of a light chain variable region (VL) and a heavy chain variable region (VH) connected to one another directly or via a peptide linker sequence.

The antigen-binding domain may specifically bind to the antigen, for example bind to the antigen but not bind to other peptides, or bind at a lower affinity to other peptides.

The binding affinity between two molecules (e.g. an antigen-binding domain and an antigen) may be quantified, for example, by determination of the dissociation constant (K_{D}). The K_{D} can be determined by measurement of the kinetics of complex formation and dissociation between the antigen-binding domain and antigen, e.g. using a technique such as surface plasmon resonance (SPR). The rate constants corresponding to the association and the dissociation of a complex are referred to as the association rate constant kₐ (or kₒₙ) and dissociation rate constant k_{d} (or k_{off}), respectively. K_{D} is related to kₐ and k_{d} through the equation K_{D} = k_{d} / kₐ.

Suitably, the antigen-binding domain is a CD84-binding domain.

| **SEQ ID NO.** | **Description** | **Sequence** |
|---|---|---|
| 1 | 152-1D5 CDR-H1 | NYWIN |
| 2 | 152-1D5 CDR-H2 | DIYPVSGTTNYNEKFKR |
| 3 | 152-1D5 CDR-H3 | GTGRFAY |
| 4 | 152-1D5 CDR-L1 | RASQSVSTSSYSYMH |
| 5 | 152-1D5 CDR-L2 | FASNLES |
| 6 | 152-1D5 CDR-L3 | QHSWEIPYT |
| 7 | 153-4D9 CDR-H1 | NYWLG |
| 8 | 153-4D9 CDR-H2 | DIYPGGGYTNYIEKFKG |
| 9 | 153-4D9 CDR-H3 | YEGGYYGNYDAMDY |
| 10 | 153-4D9 CDR-L1 | RASESVDNYGISFMN |
| 11 | 153-4D9 CDR-L2 | AASNQGS |
| 12 | 153-4D9 CDR-L3 | QQSKAVPRT |
| 13 | R3-B3 CDR-H1 | GFTFSSYA |
| 14 | R3-B3 CDR-H2 | ISGSGGST |
| 15 | R3-B3 CDR-H3 | AKWDCSDGRCYWAY |
| 16 | R3-B3 CDR-L1 | NIESKD |
| 17 | R3-B3 CDR-L2 | DDA |
| 18 | R3-B3 CDR-L3 | QVWDSSSDHVV |
| 19 | R3-G7 CDR-H1 | GFTFSSYP |
| 20 | R3-G7 CDR-H2 | ISYHGRNK |
| 21 | R3-G7 CDR-H3 | ARDRDATPGGTGVGNHGMAV |
| 22 | R3-G7 CDR-L1 | QSLLHSSGYNY |
| 23 | R3-G7 CDR-L2 | MGS |
| 24 | R3-G7 CDR-L3 | MQGLQTPPT |
| 25 | R3-H3 CDR-H1 | GFTFSDNA |
| 26 | R3-H3 CDR-H2 | ISGTGRTT |
| 27 | R3-H3 CDR-H3 | AKWDCSDGRCYWAY |
| 28 | R3-H3 CDR-L1 | QSLVYSDGDTY |
| 29 | R3-H3 CDR-L2 | KVS |
| 30 | R3-H3 CDR-L3 | MQGTHWPPNT |
| 31 | GYCD84.1-7 CDR-H1 | TSGMGVG |
| 32 | GYCD84.1-7 CDR-H2 | HIWWDDVKRYNPALKS |
| 33 | GYCD84.1-7 CDR-H3 | MRTSYYFDY |
| 34 | GYCD84.1-7 CDR-L1 | RASENIFSSLA |
| 35 | GYCD84.1-7 CDR-L2 | NAKTLAE |
| 36 | GYCD84.1-7 CDR-L3 | QHHYATPFT |
| 37 | GYCD84.1-97 CDR-H1 | SYWIN |
| 38 | GYCD84.1-97 CDR-H2 | DIYLGSGSTNYNEKFKS |
| 39 | GYCD84.1-97 CDR-H3 | SGGYLGY |
| 40 | GYCD84.1-97 CDR-L1 | RASQSVSTSSYSYMH |
| 41 | GYCD84.1-97 CDR-L2 | FASNLES |
| 42 | GYCD84.1-97 CDR-L3 | QHSWEIPYT |
| 43 | GYCD84.1-108 CDR-H1 | NYWIG |
| 44 | GYCD84.1-108 CDR-H2 | DIYPGGGYTNYNENFKG |
| 45 | GYCD84.1-108 CDR-H3 | STTYYSSYWCFDV |
| 46 | GYCD84.1-108 CDR-L1 | KSSQSLLNSGNQANYLA |
| 47 | GYCD84.1-108 | GASTRES |
| | CDR-L2 | |
| 48 | GYCD84.1-108 CDR-L3 | QNDHSYPFT |
| 49 | GYCD84.1-207 CDR-H1 | RYWMS |
| 50 | GYCD84.1-207 CDR-H2 | EINPDSSTINYTPSLKD |
| 51 | GYCD84.1-207 CDR-H3 | PGPTVVATYWYFDV |
| 52 | GYCD84.1-207 CDR-L1 | RSSQSIVHSNGNTYLE |
| 53 | GYCD84.1-207 CDR-L2 | KVSSRFS |
| 54 | GYCD84.1-207 CDR-L3 | FQGSHVPRT |
| 55 | GYCD84.1-226 CDR-H1 | RYWIN |
| 56 | GYCD84.1-226 CDR-H2 | DIYPGSGSTNYNEKFKS |
| 57 | GYCD84.1-226 CDR-H3 | DTTIAY |
| 58 | GYCD84.1-226 CDR-L1 | RASQSVTTSRYSYMH |
| 59 | GYCD84.1-226 CDR-L2 | FASNLES |
| 60 | GYCD84.1-226 CDR-L3 | QHSWEIPYT |
| 61 | 152-1D5 VH | QVQLQQPGAEVVQPGTSVKLSCKASGYNFTNYWINWVKLRPGQGLEWIGD IYPVSGTTNYNEKFKRKATLTVDTSSSTGNMQLSSLASEDSALYYCASGT GRFAYWGQGTLVTVSA |
| 62 | 152-1D5 VL | DIVLTQSPASLAVSLGQRATISCRASQSVSTSSYSYMHWYQQKPGQPPKL LIKFASNLESGVPARFSGSGSGTDFTLNIHPVEEEDTATYYCQHSWEIPY TFGGGTKLEIK |
| 63 | 153-4D9 VH | QVQLQQSGAELVRPGTSVKISCKASGYTFTNYWLGWVKQRPGHGLEWIGD IYPGGGYTNYIEKFKGKATLTADTSSSTAYMQLSSLTSEDSAVYFCARYE GGYYGNYDAMDYWGQGTSVTVSS |
| 64 | 153-4D9 VL | DIVLTQSPGSLAVSLGQRATISCRASESVDNYGISFMNWFQQKPGQPPKL LIYAASNQGSGVPARFSGSGSGTDFSLNIHPMEEDDTAMYFCQQSKAVPR TFGGGTKLEIK |
| 65 | R3-B3 VH | QVQLVESGGGLVQPGGSLRLSCAASGFTFSSYAMSWVRQAPGKGLEWVSA ISGSGGSTYYADSVKGRFTISRDNSKNTLYLQMNSLRAEDTAVYYCAKWD CSDGRCYWAYWGQGTLVTVSS |
| 66 | R3-B3 VL | QPVLTQPPSVSVAPGQTARISCGGDNIESKDVHWYQRKSGQAPVLVVHDD ADRPTGIPDRFSGSNSGNTATLTISRVEAGDEADYYCQVWDSSSDHVVFG GGTQLTVL |
| 67 | R3-B3 Linker | GGGGSGGGGSGGGGSGGGGAS |
| 68 | R3-G7 VH | QVQLVESGGGVVQPGRSLRLSCTASGFTFSSYPMHWVRQAPGKGLEWVAA ISYHGRNKFYADSVKGRFTISRDDLRSTLYLQMNNLKADDTAVYSCARDR DATPGGTGVGNHGMAVWGQGTTVTVSS |
| 69 | R3-G7 VL | EIVLTQSPLSLPVTPGEPASISCKSSQSLLHSSGYNYLDWYLQKPGQSPQ LLIHMGSNRASGVPARFSGSGSGTDFTLRISRVEAEDVGVYYCMQGLQTP PTFGGGTKLEIK |
| 70 | R3-G7 Linker | GGGGSGGGGSGGGGSGGGGAS |
| 71 | R3-H3 VH | EVQLVESGGGLVQPGGSLRLSCAASGFTFSDNAMSWVRQAPGKGLEWVSA ISGTGRTTYYADSVKGRFTISRDNSKNTLYLQMNSLRAEDTAVYYCAKWD CSDGRCYWAYWGQGTLVTVSS |
| 72 | R3-H3 VL | EIVLTQSPLSLPVTLGQPASISCRSSQSLVYSDGDTYLNWFQQRPGQSPR RLIYKVSNRDSGVPDRFSGSGSGTDFTLKISRVEAEDVGVYYCMQGTHWP PNTFGQGTRLEIK |
| 73 | R3-H3 Linker | GGGGSGGGGSGGGGSGGGGAS |
| 74 | GYCD84.1-7 VH | QVTLKESGPGILQPSQTLSLTCSFSGFSLSTSGMGVGWIRQPSGKGLEWL AHIWWDDVKRYNPALKSRLTISKDTSSSQVFLKIASVDTADTATYYCARM RTSYYFDYWGQGTTLTVSS |
| 75 | GYCD84.1-7 VL | DIQMTQSPASLSASVGETVTITCRASENIFSSLAWYQQRQGKSPQLLVYN AKTLAEGVPSRFSGSGSGTQFSLKINSLQPEDFGSYYCQHHYATPFTFGS GTKLEIK |
| 76 | GYCD84.1-97 VH | QVQLQQPGAELVKPGTSVKLSCKASGYNFTSYWINWVKLRPGQGLEWIGD IYLGSGSTNYNEKFKSKATLTVDTSSSSAYMQLSSLVSEDSALYYCATSG GYLGYWGQGTTLTVSS |
| 77 | GYCD84.1-97 VL | DIVLTQSPASLAVSLGQRATISCRASQSVSTSSYSYMHWYQQKPGQPPKL LIKFASNLESGVPARFSGSGSGTDFTLNIHPVEEEDTATYYCQHSWEIPY TFGGGTKLEIK |
| 78 | GYCD84.1-108 VH | QVQLQQSGAELVRPGTSVKMSCKAAGYTFTNYWIGWVRQRPGHGLEWIGD IYPGGGYTNYNENFKGKATLTADTSSSTAYMQLSSLTSEDSAIYYCARST TYYSSYWCFDVWGPGTTVTVSS |
| 79 | GYCD84.1-108 VL | DIVMTQSPSSLSVSAGEKVTMSCKSSQSLLNSGNQANYLAWYQQKPGQPP KLLINGASTRESGVPDRFTGSGSGTDFTLTISSVQAEDLAVYYCQNDHSY PFTFGSGTKLEIK |
| 80 | GYCD84.1-207 VH | EVKLLESGGGLVQPGGSLKLSCAASGFDFSRYWMSWVRQAPGKGLEWIGE INPDSSTINYTPSLKDKFIISRDNAKNTLYLQMSKVRSEDTALYYCARPG PTVVATYWYFDVWGAGTTVTVSS |
| 81 | GYCD84.1-207 VL | DVLMTQTPLSLPVSLGDQASISCRSSQSIVHSNGNTYLEWYLQKPGQSPK LLIYKVSSRFSGVPDRFSGSASGTDFTLKISRVEAEDLGVYYCFQGSHVP RTFGGGTKLEIK |
| 82 | GYCD84.1-226 VH | QVQLQQPGAELVKPGTSVKLSCKASGYNFTRYWINWVKLRPGQGLEWIGD IYPGSGSTNYNEKFKSKATLTVDTSSSTAYMQLSRLASEDSALYYCARDT TIAYWGQGTLVTVSA |
| 83 | GYCD84.1-226 VL | DIVLTQSPTSLAVSLGQRATISCRASQSVTTSRYSYMHWYQQKPGQPPKL LIKFASNLESGVPARFSGSGSGTDFTLNIHPVEEEDTATYYCQHSWEIPY TFGGGTKLEIK |
| 131 | GYCD84.2.32 CDR-H1 | GYNMN |
| 132 | GYCD84.2.32 CDR-H2 | NIDPYYGGTNYNQKFKG |
| 133 | GYCD84.2.32 CDR-H3 | GLLSGSFPY |
| 134 | GYCD84.2.32 CDR-L1 | RASENIYSYLA |
| 135 | GYCD84.2.32 CDR-L2 | NAKTLAE |
| 136 | GYCD84.2.32 CDR-L3 | QHHYGSPLT |
| 137 | GYCD84.3.89 CDR-H1 | RSWMS |
| 138 | GYCD84.3.89 CDR-H2 | EINPDSSTINYTPSLKD |
| 139 | GYCD84.3.89 CDR-H3 | FYDGYSIYWYFDV |
| 140 | GYCD84.3.89 CDR-L1 | RSSQSIVHSNGDTYLE |
| 141 | GYCD84.3.89 CDR-L2 | KVSNRFS |
| 142 | GYCD84.3.89 CDR-L3 | FQGSHVPRT |
| 143 | GYCD84.3.29 8 CDR-H1 | TSGMGVG |
| 144 | GYCD84.3.29 8 CDR-H2 | HIWWDDVKRYNPALRS |
| 145 | GYCD84.3.29 8 CDR-H3 | IAVTYFFDF |
| 146 | GYCD84.3.29 8 CDR-L1 | RASENIFSSFA |
| 147 | GYCD84.3.29 8 CDR-L2 | NARTLAE |
| 148 | GYCD84.3.29 8 CDR-L3 | QHHYASPFT |
| 149 | GYCD84.2.27 CDR-H1 | TSGMGVG |
| 150 | GYCD84.2.27 CDR-H2 | HIWWDDVKRYNPALKS |
| 151 | GYCD84.2.27 CDR-H3 | MSTSYYFDY |
| 152 | GYCD84.2.27 CDR-L1 | KASQSLFTSVA |
| 153 | GYCD84.2.27 CDR-L2 | SASYRYT |
| 154 | GYCD84.2.27 CDR-L3 | QQHYSSPFT |
| 155 | GYCD84.5.17 1 CDR-H1 | IYAMN |
| 156 | GYCD84.5.17 1 CDR-H2 | RIRSKSNNYARFYADSVKD |
| 157 | GYCD84.5.17 1 CDR-H3 | PLRSYFSMDY |
| 158 | GYCD84.5.17 1 CDR-L1 | KASENVDTYVS |
| 159 | GYCD84.5.17 1 CDR-L2 | GASNRYT |
| 160 | GYCD84.5.17 1 CDR-L3 | GQTYSYPWT |
| 161 | GYCD84.2.32 VH | EVQLQQSGPELEKPGASVKISCKASGYSFTGYNMNWVKQSNGKSLEWIGN IDPYYGGTNYNQKFKGKATLTVDKSSSTAYMQLKSLTSEDSAVYYCARGL LSGSFPYWGQGTLVTVSA |
| 162 | GYCD84.2.32 VL | DIQMTQSPASLSASVGETVTITCRASENIYSYLAWYQQKQGKSPQLLVYN AKTLAEGVPSRFSGSGSGTQFSLKINSLHPEDFGSYFCQHHYGSPLTFGA GTKLEVK |
| 163 | GYCD84.3.89 VH | EVKLLESGGGLVQPGGSLKLSCAASGFGFSRSWMSWVRQAPGKGLEWIGE INPDSSTINYTPSLKDKFIISRDNAKNTLFLQMSKVRSEDTALYYCATFY DGYSIYWYFDVWGAGTTVTVSS |
| 164 | GYCD84.3.89 VL | DVLMTQTPLSLPVSLGDQASISCRSSQSIVHSNGDTYLEWYLQKPGQSPK LLIYKVSNRFSGVPDRFSGSGSGTDFTLKISRVEAEDLGVYYCFQGSHVP RTFGGGTKLEIK |
| 165 | GYCD84.3.29 8 VH | QVTLKESGPGILQPSQTLSLTCSFSGFSLSTSGMGVGWIRQPSGKGLEWL AHIWWDDVKRYNPALRSRLTISKDTSSSQVFLNIASVDTADTATYYCTRI AVTYFFDFWGQGTTLTVSS |
| 166 | GYCD84.3.29 8 VL | DIQMTQSPASLSASVGETVTITCRASENIFSSFAWYQQKRGRSPQLLVYN ARTLAEGVPSRFSGSGSGTQFSLKINSLQPEDFGTYFCQHHYASPFTFGS GTELGVK |
| 167 | GYCD84.2.27 VH | QVTLKESGPGILQPSQTLSLTCSFSGFSLTTSGMGVGWIRQPSGKGLEWL AHIWWDDVKRYNPALKSRLTISKDTSSSQVFLKIASVDTADTASYYCARM STSYYFDYWGQGTTLTVSS |
| 168 | GYCD84.2.27 VL | DIVMTQSHKFMSTSLGDRVSISCKASQSLFTSVAWYQQKPGQSPKLLIYS ASYRYTGVPDRFTGSGSGTDFTFTISSVQAEDLAVYYCQQHYSSPFTFGS GTKLGIK |
| 169 | GYCD84.5.17 1 VH | EVQLVESGGGLVQPKGSLKLACAASGFTFNIYAMNWVRQAPGKGLEWVAR IRSKSNNYARFYADSVKDRFTISRDDSQSMLYLQMNNLKTEDTAMYYCVR PLRSYFSMDYWGQGTSVTVSS |
| 170 | GYCD84.5.17 1 VL | SIVMTQSPKSMSMSVGERVTLSCKASENVDTYVSWYQQKPEQSPKLLIYG ASNRYTGVPDRFTGSGSATDFTLTISSVKAEDLADYHCGQTYSYPWTFGG GTKLEIK |

In some embodiments, the antigen-binding domain comprises a heavy chain variable region (VH) having complementarity determining regions (CDRs) with the sequences: CDR1 - NYWIN (SEQ ID NO: 1); CDR2 - DIYPVSGTTNYNEKFKR (SEQ ID NO: 2); and CDR3 - GTGRFAY (SEQ ID NO: 3); or variants thereof each having up to three amino acid substitutions, additions or deletions; and a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6); or variants thereof each having up to three amino acid substitutions, additions or deletions.

In some embodiments, the antigen-binding domain comprises a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9), or variants thereof each having up to three amino acid substitutions, additions or deletions; and a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12), or variants thereof each having up to three amino acid substitutions, additions or deletions.

In some embodiments, the antigen-binding domain comprises a heavy chain variable region (VH) having complementarity determining regions (CDRs) with the sequences: CDR1 - GFTFSSYA (SEQ ID NO: 13); CDR2 - ISGSGGST (SEQ ID NO: 14); and CDR3 - AKWDCSDGRCYWAY (SEQ ID NO: 15), or variants thereof each having up to three amino acid substitutions, additions or deletions; and a light chain variable region (VL) having CDRs with the sequences: CDR1 - NIESKD (SEQ ID NO: 16); CDR2 - DDA (SEQ ID NO: 17); and CDR3 - QVWDSSSDHVV (SEQ ID NO: 18), or variants thereof each having up to three amino acid substitutions, additions or deletions.

In some embodiments, the antigen-binding domain comprises a heavy chain variable region (VH) having complementarity determining regions (CDRs) with the sequences: CDR1 - GFTFSSYP (SEQ ID NO: 19); CDR2 - ISYHGRNK (SEQ ID NO: 20); and CDR3 - ARDRDATPGGTGVGNHGMAV (SEQ ID NO: 21), or variants thereof each having up to three amino acid substitutions, additions or deletions; and a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24), or variants thereof each having up to three amino acid substitutions, additions or deletions.

In some embodiments, the antigen-binding domain comprises a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3 - AKWDCSDGRCYWAY (SEQ ID NO: 27), or variants thereof each having up to three amino acid substitutions, additions or deletions; and a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30), or variants thereof each having up to three amino acid substitutions, additions or deletions.

In some embodiments, the antigen-binding domain comprises a heavy chain variable region (VH) having complementarity determining regions (CDRs) with the sequences: CDR1 - TSGMGVG (SEQ ID NO: 31); CDR2 - HIWWDDVKRYNPALKS (SEQ ID NO: 32); and CDR3 - MRTSYYFDY (SEQ ID NO: 33), or variants thereof each having up to three amino acid substitutions, additions or deletions; and a light chain variable region (VL) having CDRs with the sequences: CDR1 - RASENIFSSLA (SEQ ID NO: 34); CDR2 - NAKTLAE (SEQ ID NO: 35); and CDR3 - QHHYATPFT (SEQ ID NO: 36), or variants thereof each having up to three amino acid substitutions, additions or deletions.

In some embodiments, the antigen-binding domain comprises a heavy chain variable region (VH) having complementarity determining regions (CDRs) with the sequences: CDR1 - SYWIN (SEQ ID NO: 37); CDR2 - DIYLGSGSTNYNEKFKS (SEQ ID NO: 38); and CDR3 - SGGYLGY (SEQ ID NO: 39), or variants thereof each having up to three amino acid substitutions, additions or deletions; and a light chain variable region (VL) having CDRs with the sequences: CDR1 - RASQSVSTSSYSYMH (SEQ ID NO: 40); CDR2 - FASNLES (SEQ ID NO: 41); and CDR3 - QHSWEIPYT (SEQ ID NO: 42), or variants thereof each having up to three amino acid substitutions, additions or deletions.

In some embodiments, the antigen-binding domain comprises a heavy chain variable region (VH) having complementarity determining regions (CDRs) with the sequences: CDR1 - NYWIG (SEQ ID NO: 43); CDR2 - DIYPGGGYTNYNENFKG (SEQ ID NO: 44); and CDR3 - STTYYSSYWCFDV (SEQ ID NO: 45), or variants thereof each having up to three amino acid substitutions, additions or deletions; and a light chain variable region (VL) having CDRs with the sequences: CDR1 - KSSQSLLNSGNQANYLA (SEQ ID NO: 46); CDR2 - GASTRES (SEQ ID NO: 47); and CDR3- QNDHSYPFT (SEQ ID NO: 48), or variants thereof each having up to three amino acid substitutions, additions or deletions.

In some embodiments, the antigen-binding domain comprises a heavy chain variable region (VH) having complementarity determining regions (CDRs) with the sequences: CDR1 - RYWMS (SEQ ID NO: 49); CDR2 - EINPDSSTINYTPSLKD (SEQ ID NO: 50); and CDR3 - PGPTVVATYWYFDV (SEQ ID NO: 51), or variants thereof each having up to three amino acid substitutions, additions or deletions; and a light chain variable region (VL) having CDRs with the sequences: CDR1 - RSSQSIVHSNGNTYLE (SEQ ID NO: 52); CDR2 - KVSSRFS (SEQ ID NO: 53); and CDR3 - FQGSHVPRT (SEQ ID NO: 54), or variants thereof each having up to three amino acid substitutions, additions or deletions.

In some embodiments, the antigen-binding domain comprises a heavy chain variable region (VH) having complementarity determining regions (CDRs) with the sequences: CDR1 - RYWIN (SEQ ID NO: 55); CDR2 - DIYPGSGSTNYNEKFKS (SEQ ID NO: 56); and CDR3 - DTTIAY (SEQ ID NO: 57), or variants thereof each having up to three amino acid substitutions, additions or deletions; and a light chain variable region (VL) having CDRs with the sequences: CDR1 - RASQSVTTSRYSYMH (SEQ ID NO: 58); CDR2 - FASNLES (SEQ ID NO: 59); and CDR3 - QHSWEIPYT (SEQ ID NO: 60), or variants thereof each having up to three amino acid substitutions, additions or deletions.

In some embodiments, the antigen-binding domain comprises a heavy chain variable region (VH) having complementarity determining regions (CDRs) with the sequences: CDR1 - GYNMN (SEQ ID NO: 131); CDR2 - NIDPYYGGTNYNQKFKG (SEQ ID NO: 132); and CDR3 - GLLSGSFPY (SEQ ID NO: 133), or variants thereof each having up to three amino acid substitutions, additions or deletions; and a light chain variable region (VL) having CDRs with the sequences: CDR1 - RASENIYSYLA (SEQ ID NO: 134); CDR2 - NAKTLAE (SEQ ID NO: 135); and CDR3 - QHHYGSPLT (SEQ ID NO: 136), or variants thereof each having up to three amino acid substitutions, additions or deletions.

In some embodiments, the antigen-binding domain comprises a heavy chain variable region (VH) having complementarity determining regions (CDRs) with the sequences: CDR1 - RSWMS (SEQ ID NO: 137); CDR2 - EINPDSSTINYTPSLKD (SEQ ID NO: 138); and CDR3 - FYDGYSIYWYFDV (SEQ ID NO: 139), or variants thereof each having up to three amino acid substitutions, additions or deletions; and a light chain variable region (VL) having CDRs with the sequences: CDR1 - RSSQSIVHSNGDTYLE (SEQ ID NO: 140); CDR2 - KVSNRFS (SEQ ID NO: 141); and CDR3 - FQGSHVPRT (SEQ ID NO: 142), or variants thereof each having up to three amino acid substitutions, additions or deletions.

In some embodiments, the antigen-binding domain comprises a heavy chain variable region (VH) having complementarity determining regions (CDRs) with the sequences: CDR1 - TSGMGVG (SEQ ID NO: 143); CDR2 - HIWWDDVKRYNPALRS (SEQ ID NO: 144); and CDR3 - IAVTYFFDF (SEQ ID NO: 145), or variants thereof each having up to three amino acid substitutions, additions or deletions; and a light chain variable region (VL) having CDRs with the sequences: CDR1 - RASENIFSSFA (SEQ ID NO: 146); CDR2 - NARTLAE (SEQ ID NO: 147); and CDR3 - QHHYASPFT (SEQ ID NO: 148), or variants thereof each having up to three amino acid substitutions, additions or deletions.

In some embodiments, the antigen-binding domain comprises a heavy chain variable region (VH) having complementarity determining regions (CDRs) with the sequences: CDR1 - TSGMGVG (SEQ ID NO: 149); CDR2 - HIWWDDVKRYNPALKS (SEQ ID NO: 150); and CDR3 - MSTSYYFDY (SEQ ID NO: 151), or variants thereof each having up to three amino acid substitutions, additions or deletions; and a light chain variable region (VL) having CDRs with the sequences: CDR1 - KASQSLFTSVA (SEQ ID NO: 152); CDR2 - SASYRYT (SEQ ID NO: 153); and CDR3 - QQHYSSPFT (SEQ ID NO: 154), or variants thereof each having up to three amino acid substitutions, additions or deletions.

In some embodiments, the antigen-binding domain comprises a heavy chain variable region (VH) having complementarity determining regions (CDRs) with the sequences: CDR1 - IYAMN (SEQ ID NO: 155); CDR2 - RIRSKSNNYARFYADSVKD (SEQ ID NO: 156); and CDR3 - PLRSYFSMDY (SEQ ID NO: 157), or variants thereof each having up to three amino acid substitutions, additions or deletions; and a light chain variable region (VL) having CDRs with the sequences: CDR1 - KASENVDTYVS (SEQ ID NO: 158); CDR2 - GASNRYT (SEQ ID NO: 159); and CDR3 - GQTYSYPWT (SEQ ID NO: 160), or variants thereof each having up to three amino acid substitutions, additions or deletions.

In some embodiments, the antigen-binding domain comprises a VH domain having the sequence of SEQ ID NO: 61; and a VL domain having the sequence of SEQ ID NO: 62 or 108, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 63; and a VL domain having the sequence of SEQ ID NO: 64 or 109, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 65; and a VL domain having the sequence of SEQ ID NO: 66, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 68; and a VL domain having the sequence of SEQ ID NO: 69, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 71; and a VL domain having the sequence of SEQ ID NO: 72, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 74; and a VL domain having the sequence of SEQ ID NO: 75, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 76; and a VL domain having the sequence of SEQ ID NO: 77, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 78; and a VL domain having the sequence of SEQ ID NO: 79, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 80; and a VL domain having the sequence of SEQ ID NO: 81, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 82; and a VL domain having the sequence of SEQ ID NO: 83, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 161; and a VL domain having the sequence of SEQ ID NO: 162, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 163; and a VL domain having the sequence of SEQ ID NO: 164, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 165; and a VL domain having the sequence of SEQ ID NO: 166, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 167; and a VL domain having the sequence of SEQ ID NO: 168, or variants thereof each having at least 75%, at least 80%, at least 85%, at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 169; and a VL domain having the sequence of SEQ ID NO: 170, or variants thereof each having at least 75%, at least 80%, at least 85%, at least 90%, at least 95%, at least 96%, at least 97%, at least 98% or at least 99%, preferably at least 90%, sequence identity thereto.

The CD84-binding domain may be a scFv. A scFv may comprise the heavy variable region (VH) and light chain variable region (VL) of an antibody, connected with a short linker peptide, for example of about 10 to 25 amino acids. The scFv may be in the orientation (from N- to C-terminus) VH-VL or VL-VH. In some embodiments, the scFv is in the orientation (from N- to C-terminus) VH-VL. In some embodiments, the scFv is in the orientation (from N- to C-terminus) VL-VH.

Example linker sequences for connecting VH and VL domains include:
GGGGSGGGGSGGGGSGGGGAS (SEQ ID NO: 67)
GGGGSGGGGSGGGGS (SEQ ID NO: 110)
GGGGSGGGGSGGGGSGGGGS (SEQ ID NO: 111)

The antigen-binding domain may comprise or consist of the sequence of any one of SEQ ID NOs: 61, 63, 112-125 or 181-184, or a variant thereof having at least 75%, at least 80%, at least 85%, at least 90%, at least 95%, at least 96%, at least 97%, at least 98% or at least 99%, preferably at least 90%, sequence identity thereto.

Suitably, the variant may bind to CD84 at least as well as the corresponding antigen-binding domain shown as SEQ ID NO: 61, 63, 112-125 or 181-184. For example, the variant may specifically bind to CD84 with a binding affinity which is at least equivalent to the binding affinity between the corresponding antigen-binding domain shown as SEQ ID NO: 61, 63, 112-125 or 181-184 and CD84.

| **Antigen-binding domain** | **Sequence** |
|---|---|
| **152.1** (VH) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSA (SEQ ID NO: 61) |
| **152.2** (VLS3VH) | DIVLTQSPASLAVSLGQRATISCRASQSVSTSSYSYMHWYQQKPG QPPKLLIKFASNLESGVPARFSGSGSGTDFTLNIHPVEEEDTATY YCQHSWEIPYTFGGGTKLEIKRAGGGGSGGGGSGGGGSQVQLQQP |
| | GAEVVQPGTSVKLSCKASGYNFTNYWINWVKLRPGQGLEWIGDIY PVSGTTNYNEKFKRKATLTVDTSSSTGNMQLSSLASEDSALYYCA SGTGRFAYWGQGTLVTVSA (SEQ ID NO: 112) |
| **152.3** (VLS4VH) | DIVLTQSPASLAVSLGQRATISCRASQSVSTSSYSYMHWYQQKPG QPPKLLIKFASNLESGVPARFSGSGSGTDFTLNIHPVEEEDTATY YCQHSWEIPYTFGGGTKLEIKRAGGGGSGGGGSGGGGSGGGGSQV QLQQPGAEVVQPGTSVKLSCKASGYNFTNYWINWVKLRPGQGLEW IGDIYPVSGTTNYNEKFKRKATLTVDTSSSTGNMQLSSLASEDSA LYYCASGTGRFAYWGQGTLVTVSA (SEQ ID NO: 113) |
| **152.4** (VHS3VL) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSAGGGGSGGGGSGGGGSDIVL TQSPASLAVSLGQRATISCRASQSVSTSSYSYMHWYQQKPGQPPK LLIKFASNLESGVPARFSGSGSGTDFTLNIHPVEEEDTATYYCQH SWEIPYTFGGGTKLEIKRA (SEQ ID NO: 114) |
| **152.5** (VHS4VL) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSAGGGGSGGGGSGGGGSGGGG SDIVLTQSPASLAVSLGQRATISCRASQSVSTSSYSYMHWYQQKP GQPPKLLIKFASNLESGVPARFSGSGSGTDFTLNIHPVEEEDTAT YYCQHSWEIPYTFGGGTKLEIKRA (SEQ ID NO: 115) |
| **153.1** (VH) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSS (SEQ ID NO: 63) |
| **153.2** (VLS3VH) | DIVLTQSPGSLAVSLGQRATISCRASESVDNYGISFMNWFQQKPG QPPKLLIYAASNQGSGVPARFSGSGSGTDFSLNIHPMEEDDTAMY FCQQSKAVPRTFGGGTKLEIKRAGGGGSGGGGSGGGGSQVQLQQS GAELVRPGTSVKISCKASGYTFTNYWLGWVKQRPGHGLEWIGDIY PGGGYTNYIEKFKGKATLTADTSSSTAYMQLSSLTSEDSAVYFCA RYEGGYYGNYDAMDYWGQGTSVTVSS (SEQ ID NO: 116) |
| **153.3** (VLS4VH) | DIVLTQSPGSLAVSLGQRATISCRASESVDNYGISFMNWFQQKPG QPPKLLIYAASNQGSGVPARFSGSGSGTDFSLNIHPMEEDDTAMY FCQQSKAVPRTFGGGTKLEIKRAGGGGSGGGGSGGGGSGGGGSQV QLQQSGAELVRPGTSVKISCKASGYTFTNYWLGWVKQRPGHGLEW IGDIYPGGGYTNYIEKFKGKATLTADTSSSTAYMQLSSLTSEDSA VYFCARYEGGYYGNYDAMDYWGQGTSVTVSS (SEQ ID NO: 117) |
| **153.4** (VHS3VL) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSSGGGGSGGGGSGG GGSDIVLTQSPGSLAVSLGQRATISCRASESVDNYGISFMNWFQQ KPGQPPKLLIYAASNQGSGVPARFSGSGSGTDFSLNIHPMEEDDT AMYFCQQSKAVPRTFGGGTKLEIKRA (SEQ ID NO: 118) |
| **153.5** (VHS4VL) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSSGGGGSGGGGSGG GGSGGGGSDIVLTQSPGSLAVSLGQRATISCRASESVDNYGISFM NWFQQKPGQPPKLLIYAASNQGSGVPARFSGSGSGTDFSLNIHPM EEDDTAMYFCQQSKAVPRTFGGGTKLEIKRA (SEQ ID NO: 119) |
| **B3.4** (VHS3VL) | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKWDCSDGRCYWAYWGQGTLVTVSSGGGGSGGGGSGGGG SQPVLTQPPSVSVAPGQTARISCGGDNIESKDVHWYQRKSGQAPV LVVHDDADRPTGIPDRFSGSNSGNTATLTISRVEAGDEADYYCQV WDSSSDHVVFGGGTQLTVL (SEQ ID NO: 120) |
| **B3.5** (VHS4VL) | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKWDCSDGRCYWAYWGQGTLVTVSSGGGGSGGGGSGGGG SGGGGSQPVLTQPPSVSVAPGQTARISCGGDNIESKDVHWYQRKS GQAPVLVVHDDADRPTGIPDRFSGSNSGNTATLTISRVEAGDEAD YYCQVWDSSSDHVVFGGGTQLTVL (SEQ ID NO: 121) |
| **G7.4** (VHS3VL) | QVQLVESGGGVVQPGRSLRLSCTASGFTFSSYPMHWVRQAPGKGL EWVAAISYHGRNKFYADSVKGRFTISRDDLRSTLYLQMNNLKADD TAVYSCARDRDATPGGTGVGNHGMAVWGQGTTVTVSSGGGGSGGG GSGGGGSEIVLTQSPLSLPVTPGEPASISCKSSQSLLHSSGYNYL DWYLQKPGQSPQLLIHMGSNRASGVPARFSGSGSGTDFTLRISRV EAEDVGVYYCMQGLQTPPTFGGGTKLEIK (SEQ ID NO: 122) |
| **G7.5** (VHS4VL) | QVQLVESGGGVVQPGRSLRLSCTASGFTFSSYPMHWVRQAPGKGL EWVAAISYHGRNKFYADSVKGRFTISRDDLRSTLYLQMNNLKADD TAVYSCARDRDATPGGTGVGNHGMAVWGQGTTVTVSSGGGGSGGG GSGGGGSGGGGSEIVLTQSPLSLPVTPGEPASISCKSSQSLLHSS GYNYLDWYLQKPGQSPQLLIHMGSNRASGVPARFSGSGSGTDFTL RISRVEAEDVGVYYCMQGLQTPPTFGGGTKLEIK (SEQ ID NO: 123) |
| **H3.4** (VHS3VL) | EVQLVESGGGLVQPGGSLRLSCAASGFTFSDNAMSWVRQAPGKGL EWVSAISGTGRTTYYADSVKGRFTISRDNSKNTLYLQMNSLRAED TAVYYCAKWDCSDGRCYWAYWGQGTLVTVSSGGGGSGGGGSGGGG SEIVLTQSPLSLPVTLGQPASISCRSSQSLVYSDGDTYLNWFQQR PGQSPRRLIYKVSNRDSGVPDRFSGSGSGTDFTLKISRVEAEDVG VYYCMQGTHWPPNTFGQGTRLEIK (SEQ ID NO: 124) |
| **H3.5** (VHS4VL) | EVQLVESGGGLVQPGGSLRLSCAASGFTFSDNAMSWVRQAPGKGL EWVSAISGTGRTTYYADSVKGRFTISRDNSKNTLYLQMNSLRAED TAVYYCAKWDCSDGRCYWAYWGQGTLVTVSSGGGGSGGGGSGGGG SGGGGSEIVLTQSPLSLPVTLGQPASISCRSSQSLVYSDGDTYLN WFQQRPGQSPRRLIYKVSNRDSGVPDRFSGSGSGTDFTLKISRVE AEDVGVYYCMQGTHWPPNTFGQGTRLEIK (SEQ ID NO: 125) |
| **1.7.3** (VLS4VH) | DIQMTQSPASLSASVGETVTITCRASENIFSSLAWYQQRQGKSPQ LLVYNAKTLAEGVPSRFSGSGSGTQFSLKINSLQPEDFGSYYCQH HYATPFTFGSGTKLEIKGGGGSGGGGSGGGGSGGGGSQVTLKESG PGILQPSQTLSLTCSFSGFSLSTSGMGVGWIRQPSGKGLEWLAHI WWDDVKRYNPALKSRLTISKDTSSSQVFLKIASVDTADTATYYCA RMRTSYYFDYWGQGTTLTVSS (SEQ ID NO: 181) |
| **1.7.5** (VHS4VL) | QVTLKESGPGILQPSQTLSLTCSFSGFSLSTSGMGVGWIRQPSGK GLEWLAHIWWDDVKRYNPALKSRLTISKDTSSSQVFLKIASVDTA DTATYYCARMRTSYYFDYWGQGTTLTVSSGGGGSGGGGSGGGGSG GGGSDIQMTQSPASLSASVGETVTITCRASENIFSSLAWYQQRQG KSPQLLVYNAKTLAEGVPSRFSGSGSGTQFSLKINSLQPEDFGSY YCQHHYATPFTFGSGTKLEIK (SEQ ID NO: 182) |
| **1.226.5** (VHS4VL) | QVQLQQPGAELVKPGTSVKLSCKASGYNFTRYWINWVKLRPGQGL EWIGDIYPGSGSTNYNEKFKSKATLTVDTSSSTAYMQLSRLASED SALYYCARDTTIAYWGQGTLVTVSAGGGGSGGGGSGGGGSGGGGS DIVLTQSPTSLAVSLGQRATISCRASQSVTTSRYSYMHWYQQKPG QPPKLLIKFASNLESGVPARFSGSGSGTDFTLNIHPVEEEDTATY YCQHSWEIPYTFGGGTKLEIK (SEQ ID NO: 183) |
| **3.89.5** (VHS4VL) | EVKLLESGGGLVQPGGSLKLSCAASGFGFSRSWMSWVRQAPGKGL EWIGEINPDSSTINYTPSLKDKFIISRDNAKNTLFLQMSKVRSED |
| | TALYYCATFYDGYSIYWYFDVWGAGTTVTVSSGGGGSGGGGSGGG GSGGGGSDVLMTQTPLSLPVSLGDQASISCRSSQSIVHSNGDTYL EWYLQKPGQSPKLLIYKVSNRFSGVPDRFSGSGSGTDFTLKISRV EAEDLGVYYCFQGSHVPRTFGGGTKLEIK (SEQ ID NO: 184) |

In some embodiments, the antigen-binding domain comprises or consists of the sequence of SEQ ID NO: 61, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 63, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 112, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 113, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 114, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 115, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 116, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 117, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 118, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 119, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 120, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 121, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 122, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 123, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 124, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 125, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 181, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 182, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 183, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 184, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

The antigen-binding domain may be comprised in a chimeric antigen receptor (CAR).

### Chimeric antigen receptor (CAR)

"Chimeric antigen receptor" (CAR or CARs) as used herein refers to engineered receptors which can confer an antigen specificity onto cells (for example, T cells, such as naive T cells, central memory T cells, effector memory T cells or combinations thereof). CARs are also known as artificial T-cell receptors, chimeric T-cell receptors or chimeric immunoreceptors. CARs can also confer an antigen specificity onto other immune cells such as NK cells (e.g. from cord blood (CB), induced pluripotent stem cells (iPSCs), bone marrow (BM), human embryonic stem cells (hESCs), a cell line (e.g. NK92 or YT), or peripheral blood (PB) (Mehta et al. (2018) Front. Immunol. 9: 283; Liu et al. (2020) N. Engl. J. Med. 382: 545-553). CARs used in NK cells may have other transmembrane domains (such as NKG2D or DAP12) and other co-stimulatory domains (such as NKG2D or 2B4) and they may incorporate genes for IL-2 or IL-15 within the CAR construct to constantly provide cytokine support to the CAR-NK cells.

CARs may, for example, comprise an antigen-binding domain, a transmembrane domain and an intracellular signaling domain (endodomain).

In some embodiments, the CAR comprises a CD84-binding domain, a transmembrane domain and an intracellular signaling domain. The CAR may comprise one or more co-stimulatory domain.

The antigen-binding domain (e.g. CD84-binding domain) of the CAR may be an antigen-binding domain as disclosed herein.

In another aspect, the invention provides a chimeric antigen receptor (CAR) comprising or consisting of the sequence of any one of SEQ ID NOs: 172-180, or a variant thereof having at least 75%, at least 80%, at least 85%, at least 90%, at least 95%, at least 96%, at least 97%, at least 98% or at least 99%, preferably at least 90%, sequence identity thereto.

Suitably, the variant may function at least as well as the corresponding CAR shown as SEQ ID NO: 172-180. For example, the variant may specifically bind to CD84 with a binding affinity which is at least equivalent to the binding affinity between the corresponding CAR shown as SEQ ID NO: 172-180 and CD84.

In another aspect, the invention provides a chimeric antigen receptor (CAR) comprising or consisting of the sequence of SEQ ID NO: 172, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 173, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 174, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 175, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 176, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 177, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 178, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 179, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 180, or a variant thereof having at least 75%, at least 80%, at least 85%, at least 90%, at least 95%, at least 96%, at least 97%, at least 98% or at least 99%, preferably at least 90%, sequence identity thereto.

### Transmembrane domain

The CAR may comprise a transmembrane domain.

The transmembrane domain may comprise the transmembrane sequence from any protein which has a transmembrane domain, including any of the type I, type II or type III transmembrane proteins. The transmembrane domain of the CAR may also comprise an artificial hydrophobic sequence. The transmembrane domains of the CAR may be selected so as not to dimerise.

Examples of transmembrane (TM) regions used in CAR constructs are: a) the CD28 TM region (Pulè et al., Mol Ther, 2005, Nov;12(5):933-41; Brentjens et al., CCR, 2007, Sep 15; 13(18 Pt 1):5426-35; Casucci et al., Blood, 2013, Nov 14;122(20):3461-72.); b) the OX40 TM region (Pulè et al., Mol Ther, 2005, Nov;12(5):933-41); c) the 4-1BB TM region (Brentjens et al, CCR, 2007, Sep 15;13 (18 Pt 1):5426-35); d) the CD3-zeta TM region (Pulè et al., Mol Ther, 2005, Nov;12(5):933-41; Di Stasi et al. Blood, 2009, Jun 18;113(25):6392-402.); e) the CD8a TM region (Maher et al., Nat Biotechnol, 2002, Jan;20(1):70-5.; Imai C et al., Leukemia, 2004, Apr;18(4):676-84; Brentjens et al., CCR, 2007, Sep 15;13(18 Pt 1):5426-35; Milone et al., Mol Ther, 2009, Aug;17(8):1453-64.); f) the DAP12 TM region (Müller, N. et al. J. Immunother. 2015, June 01; 38, 197); g) the 2B4 TM region (Altvater, B. et al. Clin. Cancer Res, 2009, July 22; (15) 4857-4866) and h) the NKG2D TM region (Li, Y et al, Cell Stem Cell, 2018, Aug 2; (23):181-192).

Additional transmembrane domains will be apparent to those of skill in the art.

An example amino acid sequence of a CD8a transmembrane domain is:
IYIWAPLAGTCGVLLLSLVITLYC (SEQ ID NO: 127)

An example amino acid sequence of a CD28 transmembrane domain is:
FWVLVVVGGVLACYSLLVTVAFIIFWV (SEQ ID NO: 128)

In some embodiments, the transmembrane domain comprises or consists of the sequence of SEQ ID NO: 127 or 128, or a variant thereof having at least 75%, at least 80%, at least 85%, at least 90%, at least 95%, at least 96%, at least 97%, at least 98% or at least 99%, preferably at least 90%, sequence identity thereto.

### Signal peptide

The CAR may comprise a signal peptide such that when the CAR is expressed in a cell the protein is directed to the endoplasmic reticulum and subsequently to the cell surface.

Signal peptides may be recognised and cleaved by a signal peptidase during or after translocation to generate a mature protein.

In some embodiments, the signal peptide is a CD8a signal peptide.

### Spacer

The CAR may comprise a spacer that joins the antigen-binding domain to the transmembrane domain.

The spacer sequence may, for example, comprise an IgG1 Fc region, an IgG1 hinge or a human or mouse CD8 stalk.

In some embodiments, the CAR comprises a CD8a stalk.

### Intracellular signalling domain

The intracellular domain may provide for signal-transmission in a CAR.

The intracellular signalling domain may comprise one or more immunoreceptor tyrosine-based activation motif (ITAM), which is a conserved sequence of four amino acids repeated twice in the cytoplasmic tails of certain cell-surface proteins of the immune system. The motif contains a tyrosine separated from a leucine or isoleucine by any two other amino acids (YxxL/I). The tyrosine residues of these motifs may be phosphorylated following interaction of the receptor molecules with their ligands and may form binding sites for other proteins involved in signalling pathways.

The intracellular signalling domain may comprise or consist of the CD3-zeta endodomain, which contains three ITAMs. The CD3-zeta endodomain may transmit an activation signal to the T cell after the antigen is bound.

The CAR may comprise one or more co-stimulatory domain. For example, 4-1BB (also known as CD137) can be used with CD3-zeta, or CD28, OX40 and/or ICOS can be used with CD3-zeta to transmit a proliferative / survival signal. Also NKG2D, 2B4 (also known as CD244), DAP12 and/or DAP10 can be used with CD3-zeta to transmit a proliferative / survival signal.

In some embodiments, the CAR comprises a CD3-zeta signalling domain and lacks any co-stimulatory domains. In some embodiments, the CAR comprises a CD3-zeta signalling domain and one or more co-stimulatory domain.

In some embodiments, the CAR comprises one or more co-stimulatory domains selected from the group consisting of a 4-1BB co-stimulatory domain, a CD28 co-stimulatory domain and an OX40 co-stimulatory domain.

In preferred embodiments, the CAR comprises a 4-1BB co-stimulatory domain. In some embodiments, the CAR comprises a CD28 co-stimulatory domain. In some embodiments, the CAR comprises a OX40 co-stimulatory domain.

In preferred embodiments, the CAR comprises a 4-1BB co-stimulatory domain and a CD3-zeta signalling domain.

An example amino acid sequence of a CD3-zeta signalling domain is:

RVKFSRSADAPAYQQGQNQLYNELNLGRREEYDVLDKRRGRDPEMGGKPQRRKNPQEGLYNE
LQKDKMAEAYSEIGMKGERRRGKGHDGLYQGLSTATKDTYDALHMQALPPR

(SEQ ID NO: 129)

In some embodiments, the signalling domain comprises or consists of the sequence of SEQ ID NO: 129, or a variant thereof having at least 75%, at least 80%, at least 85%, at least 90%, at least 95%, at least 96%, at least 97%, at least 98% or at least 99%, preferably at least 90%, sequence identity thereto.

An example amino acid sequence of a 4-1BB co-stimulatory domain is:
KRGRKKLLYIFKQPFMRPVQTTQEEDGCSCRFPEEEEGGCEL (SEQ ID NO: 130)

An example amino acid sequence of a CD28 co-stimulatory domain is:
RSKRSRLLHSDYMNMTPRRPGPTRKHQYPYAPPRDFAAYRS (SEQ ID NO: 171)

In some embodiments, the co-stimulatory domain comprises or consists of the sequence of SEQ ID NO: 130 or 171, or a variant thereof having at least 75%, at least 80%, at least 85%, at least 90%, at least 95%, at least 96%, at least 97%, at least 98% or at least 99%, preferably at least 90%, sequence identity thereto.

### Polynucleotide

Polynucleotides of the invention may comprise DNA or RNA, preferably DNA. They may be single-stranded or double-stranded. Preferably the polynucleotides are isolated polynucleotides. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The polynucleotides may be modified by any method available in the art. Such modifications may be carried out in order to enhance the *in vivo* activity or lifespan of the polynucleotides of the invention.

Polynucleotides such as DNA polynucleotides may be produced recombinantly, synthetically or by any means available to those of skill in the art. They may also be cloned by standard techniques.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing a PCR under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

The polynucleotide may comprise a promoter and/or enhancer operably linked to the one or more nucleotide sequence encoding the antigen-binding domain, antibody or CAR of the invention. The term "operably linked", as used herein, may mean that two components are linked together in a manner, which enables both to carry out their function substantially unhindered. For example, the promoter and/or enhancer may facilitate and/or enhance expression of the antigen-binding domain, antibody or CAR.

In some embodiments, the promoter is a EF1α promoter.

### Vectors

In some embodiments, the polynucleotide is a vector.

Preferably the vector is a viral vector, such as a retroviral vector, lentiviral vector, adeno-associated viral (AAV) vector or adenoviral vector. In some embodiments the polynucleotide is a viral genome.

In another aspect the invention provides a viral vector comprising the polynucleotide of the invention.

In some embodiments the viral vector is in the form of a viral vector particle.

A vector is a tool that allows or facilitates the transfer of an entity from one environment to another. In accordance with the invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid and/or facilitating the expression of the protein encoded by a segment of nucleic acid.

Vectors comprising polynucleotides used in the invention may be introduced into cells using a variety of techniques known in the art, such as transfection, transduction and transformation.

Transfection may refer to a general process of incorporating a nucleic acid into a cell and includes a process using a non-viral vector to deliver a polynucleotide to a cell. Transduction may refer to a process of incorporating a nucleic acid into a cell using a viral vector.

### Retroviral and lentiviral vectors

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV). A detailed list of retroviruses may be found in Coffin, J.M. et al. (1997) Retroviruses, Cold Spring Harbour Laboratory Press, 758-63.

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

The basic structures of retrovirus and lentivirus genomes share many common features such as a 5' LTR and a 3' LTR. Between or within these are located a packaging signal to enable the genome to be packaged, a primer-binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective.

Lentivirus vectors are part of the larger group of retroviral vectors. A detailed list of lentiviruses may be found in Coffin, J.M. et al. (1997) Retroviruses, Cold Spring Harbour Laboratory Press, 758-63. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells (Lewis, P et al. (1992) EMBO J. 11: 3053-8; Lewis, P.F. et al. (1994) J. Virol. 68: 510-6). In contrast, other retroviruses, such as MLV, are unable to infect non-dividing or slowly dividing cells such as those that make up, for example, muscle, brain, lung and liver tissue.

A lentiviral vector, as used herein, is a vector, which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae, which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells *in vivo* with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site; Naldini, L. et al. (1996) Science 272: 263-7; Naldini, L. et al. (1996) Proc. Natl. Acad. Sci. USA 93: 11382-8; Leavitt, A.D. et al. (1996) J. Virol. 70: 721-8) or by modifying or deleting essential att sequences from the vector LTR (Nightingale, S.J. et al. (2006) Mol. Ther. 13: 1121-32), or by a combination of the above.

### Cells

In another aspect the invention provides a cell comprising the polynucleotide, the vector, the antigen-binding domain or the CAR of the invention.

In some embodiments the cell is a T cell, lymphocyte or stem cell, such as a hematopoietic stem cell, cord blood stem cell (CB) or induced pluripotent stem cell (iPSC).

For example, the cell may be selected from the group consisting of CD4 cells, CD8 cells, Th0 cells, Tc0 cells, Th1 cells, Tc1 cells, Th2 cells, Tc2 cells, Th17 cells, Th22 cells, gamma/delta T cells, natural killer (NK) cells, natural killer T (NKT) cells, double-negative T cells, naive T cells, memory stem T cells, central memory T cells, effector memory T cells, effector T cells, cytokine-induced killer (CIK) cells, hematopoietic stem cells and induced pluripotent stem cells (iPSC).

In some embodiments, the cell is a T cell or NK cell, preferably a T cell. In some embodiments, the T cell is an autologous or allogeneic T cell.

The cell may have been isolated from a subject.

The cell of the invention may be provided for use in adoptive cell transfer. As used herein the term "adoptive cell transfer" refers to the administration of a cell population to a patient. Typically, the cells are T cells isolated from a subject and then genetically modified and cultured *in vitro* before being administered to the patient.

Adoptive cell transfer may be allogenic or autologous.

By "autologous cell transfer" it is to be understood that the starting population of cells (which are then transduced with a polynucleotide or vector according to the invention) is obtained from the same subject as that to which the transduced cell population is administered. Autologous transfer is advantageous as it avoids problems associated with immunological incompatibility and is available to subjects irrespective of the availability of a genetically matched donor.

By "allogeneic cell transfer" it is to be understood that the starting population of cells (which are then transduced with a polynucleotide or vector according to the invention) is obtained from a different subject as that to which the transduced cell population is administered. Preferably, the donor will be genetically matched to the subject to which the cells are administered to minimise the risk of immunological incompatibility. Alternatively, the donor may be mismatched and unrelated to the patient.

### Method of treatment

In another aspect, the invention provides the antigen-binding domain, the antibody, the CAR, the polynucleotide, the vector, the cell or the pharmaceutical composition of the invention for use in therapy.

In some embodiments, the therapy is treatment of cancer.

In preferred embodiments, the cancer is a haematological malignancy.

In preferred embodiments, the cancer cells express CD84, for example the haematological malignancy may be a CD84-expressing haematological malignancy.

In some embodiments, the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

In some embodiments, the cancer is selected from the group consisting of chronic lymphocytic leukaemia (CLL), diffuse large B-cell lymphoma (DLBCL), Burkitt's lymphoma, follicular lymphoma, mantle cell lymphoma, B-cell acute lymphoblastic leukaemia (B-ALL), T-cell acute lymphoblastic leukaemia/lymphoma (T-ALL), acute myeloid leukemia (AML) and histiocytic sarcoma.

In preferred embodiments, the cancer is selected from the group consisting of CLL, B-cell lymphoma, B-ALL, T-ALL and AML.

In preferred embodiments, the cancer is selected from the group consisting of B-cell lymphoma, B-ALL, T-ALL and AML.

In some embodiments, the cancer is not a T-cell lymphoma. In some embodiments, the cancer is not a mature T-cell lymphoma. In some embodiments, the cancer is not CLL. In some embodiments, the cancer is not a solid tumour.

The treatment of mammals, particularly humans, is preferred. Both human and veterinary treatments are within the scope of the invention.

### Pharmaceutical compositions and injected solutions

Although the agents for use in the invention can be administered alone, they will generally be administered in admixture with a pharmaceutical carrier, excipient or diluent, particularly for human therapy.

The medicaments, for example cells or vector particles, of the invention may be formulated into pharmaceutical compositions. These compositions may comprise, in addition to the medicament, a pharmaceutically acceptable carrier, diluent, excipient, buffer, stabiliser or other materials well known in the art. Such materials should be non-toxic and should not interfere with the efficacy of the active ingredient. The precise nature of the carrier or other material may be determined by the skilled person according to the route of administration, e.g. intravenous or intra-arterial.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001% may be used. In some cases, serum albumin may be used in the composition.

For injection, the active ingredient may be in the form of an aqueous solution, which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

Handling of the cell therapy products is preferably performed in compliance with FACT-JACIE International Standards for cellular therapy.

### Administration

In some embodiments, the antigen-binding domain, the antibody, the CAR, the polynucleotide, the vector, the cell or the pharmaceutical composition of the invention is administered to a subject systemically.

In some embodiments, the antigen-binding domain, the antibody, the CAR, the polynucleotide, the vector, the cell or the pharmaceutical composition of the invention is administered to a subject locally.

The term "systemic delivery" or "systemic administration" as used herein means that the agent of the invention is administered into the circulatory system, for example to achieve broad distribution of the agent. In contrast, topical or local administration restricts the delivery of the agent to a localised area.

In some embodiments, the antigen-binding domain, the antibody, the CAR, the polynucleotide, the vector, the cell or the pharmaceutical composition of the invention is administered intravascularly, intravenously or intra-arterially.

In preferred embodiments, the antigen-binding domain, the antibody, the CAR, the polynucleotide, the vector, the cell or the pharmaceutical composition of the invention is administered intravenously.

### Dosage

The skilled person can readily determine an appropriate dose of an agent of the invention to administer to a subject. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can of course be individual instances where higher or lower dosage ranges are merited, and such are within the scope of the invention.

### Subject

The term "subject" as used herein refers to either a human or non-human animal.

Examples of non-human animals include vertebrates, for example mammals, such as non-human primates (particularly higher primates), dogs, rodents (e.g. mice, rats or guinea pigs), pigs and cats. The non-human animal may be a companion animal.

Preferably, the subject is human.

### Variants, derivatives, analogues, homologues and fragments

In addition to the specific proteins and nucleotides mentioned herein, the invention also encompasses variants, derivatives, analogues, homologues and fragments thereof.

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring polypeptide or polynucleotide.

The term "derivative" as used herein in relation to proteins or polypeptides of the invention includes any substitution of, variation of, modification of, replacement of, deletion of and/or addition of one (or more) amino acid residues from or to the sequence, providing that the resultant protein or polypeptide retains at least one of its endogenous functions.

The term "analogue" as used herein in relation to polypeptides or polynucleotides includes any mimetic, that is, a chemical compound that possesses at least one of the endogenous functions of the polypeptides or polynucleotides, which it mimics.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | GAP |
| | | I L V |
| | Polar - uncharged | C S T M |
| | | N Q |
| | Polar - charged | D E |
| | | K R H |
| AROMATIC | | FWY |

The term "homologue" as used herein means an entity having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package (see Ausubel *et al.* (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel *et al.* (1999) ibid, pages 7-58 to 7-60).

However, for some applications, it is preferred to use the GCG Bestfit program. Another tool, BLAST 2 Sequences, is also available for comparing protein and nucleotide sequences (FEMS Microbiol. Lett. (1999) 174: 247-50; FEMS Microbiol. Lett. (1999) 177: 187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells, as well as for a variety of other organisms.

The skilled person will understand that they can combine all features of the invention disclosed herein without departing from the scope of the invention as disclosed.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. Each of these general texts is herein incorporated by reference.

### EXAMPLES

### EXAMPLE 1: Expression of CD84 in haematological malignancies

We have observed that CD84 is overexpressed in a range of malignant haematological diseases. Using GEPIA, an interactive web server for analysing the RNA sequencing expression data of 9,736 tumours and 8,587 normal samples from the TCGA and the GTEx projects (http://gepia.cancer-pku.cn/index.html; Tang et al. GEPIA: a web server for cancer and normal gene expression profiling and interactive analyses. Nucleic Acids Res. 2017; 10.1093/nar/gkx247) we have found that:
- CD84 expression is 4.2 times higher in 47 Lymphoid Neoplasm Diffuse Large B-cell Lymphoma (in Figure 1 presented as DLBC) than in 337 blood samples.
- CD84 expression is 10.1 times higher in 173 Acute Myeloid Leukaemia (in Figure 1 presented as LAML) than in 70 bone marrow samples.

We also interrogated the Cancer Cell Line Encyclopaedia (CCLE) dataset, which contains mRNA expression data for a panel of more than 1,100 cell lines derived from different types of solid and haematological tumours (https://portals.broadinstitute.org/ccle). Both RNAseq and Affymetrix analysis of mRNA expression showed that CD84 is expressed at high levels in cell lines derived from Burkitt's lymphoma, acute myeloid leukaemia (AML), B-cell lymphomas, chronic myeloid leukaemia (CML), B-cell acute lymphoblastic leukaemia (B-ALL) and T-cell acute lymphoblastic leukaemia (T-ALL), but not in T-cell lymphomas or solid tumours (Figure 2).

We then assessed the expression of CD84 on the surface of cell lines derived from different haematological malignancies. Table 1 shows the cell lines that were used, the haematological malignancy from which they originate and a qualitative assessment of CD84 expression based on the cytometry analysis shown in Figure 3.

**Table 1. List of cells lines derived from haematological malignancies used to assess surface CD84 expression and qualitative assessment of CD84 expression.**

| **Cell Line** | **Haematological malignancy** | **CD84 expression** |
|---|---|---|
| Ramos | Burkitt's Lymphoma | High |
| Raji | Burkitt's Lymphoma | High |
| Daudi | Burkitt's Lymphoma | High |
| NALM6 | B-cell acute lymphoblastic leukaemia | High |
| MOLT-4 | T-cell acute lymphoblastic leukaemia | High |
| U937 | Histiocytic lymphoma | Low |
| Kasumi-1 | Acute myeloblastic leukaemia | Moderate |
| K562 | Chronic myelogenous leukaemia (in blast crisis) | Low |
| THP-1 | Acute monocytic leukaemia | Low |

We analysed the expression of CD84 in 9 samples from patients with CLL by flow cytometry. Table 2 shows a qualitative assessment of CD84 expression based on the cytometry analysis shown in Figure 4. The expression of CD84 on the leukemic cells was compared to the expression of CD84 in lymphocytes (CD84^{moderate}) and monocytes (CD84^{high}). The histogram in light grey represents the staining with an isotype-matched control antibody and the histogram in dark grey the staining with the specific CD84 antibody.

**Table 2. Expression of CD84 in leukemic cells from 9 patients with chronic lymphocytic leukaemia (CLL) assessed by flow cytometry.**

| **Patient number** | **CD84 expression** |
|---|---|
| P01 | Moderate |
| P02 | High |
| P03 | High |
| P04 | High |
| P05 | High |
| P06 | High |
| P07 | High |
| P08 | High |
| P09 | High |

While certain genomic databases suggest that CD84 is overexpressed at mRNA level in AML, we sought to confirm if CD84 is expressed on the surface of malignant cells from patients (n=10) diagnosed with AML (Table 3).

**Table 3. Expression of CD84 in leukemic cells of 10 patients with acute myeloid leukaemia (AML) assessed by flow cytometry.**

| **Patient number** | **CD84 expression** |
|---|---|
| P01 | Moderate |
| P02 | Moderate |
| P03 | High |
| P04 | Moderate |
| P05 | Moderate |
| P06 | Moderate |
| P07 | Moderate |
| P08 | Moderate |
| P09 | Moderate |
| P10 | High |

Two representative examples of flow cytometry data are shown in Figure 5: samples from patient 04 and patient 10 show moderate and high expression of CD84, respectively.

### EXAMPLE 2: CD84 antibodies

### Mouse monoclonal antibodies

We studied and determined the sequences of two anti-CD84 mouse monoclonal antibodies (152-1D5 and 153-4D9; Engel et al. B-cell antigens section report, in Schlossman S (ed): Leucocyte Typing V. Oxford, UK, Oxford University Press, 1995, page 483; Palou et al. Genomic characterization of CD84 reveals the existence of five isoforms differing in their cytoplasmic domains. Tissue Antigens. 2000; 55(2): 118-27).

In addition, we generated a number of new anti-CD84 mouse monoclonal antibodies as described in the Methods by immunizing BALB/c mice with the human CD84 protein. Table 3 summarizes the features of these antibodies, characterized as described in Methods. Binding to 300.19-CD84⁺, Raji and Ramos cells, lymphocytes and monocytes was carried out using hybridoma supernatants with equal amounts/concentration of anti-CD84 antibody.

**Table 4. Summary of features of anti-CD84 mouse monoclonal antibodies.**

| **Antibody** | **Class** | **Domain** | **300.19-CD84 binding** | **Raji binding** | **Ramos binding** | **Lymphocyte binding** | **Monocyte binding** |
|---|---|---|---|---|---|---|---|
| 152-1D5 | IgG1, κ | hD1 | 92.5% | 97.6% | 73.4% | 24.4% | 72.0% |
| 153-4D9 | IgG1, κ | hD1 | 88.0% | 65.8% | 34.3% | 15.9% | 47.7% |
| GYCD84.1 108 | IgG2a, κ | hD1 | 93.9% | 30.3% | 31.2% | 13.6% | 41.3% |
| GYCD84.1 226 | IgG1, κ | hD1 | 98.1% | 99.9% | 80.2% | 28.7% | 76.1% |
| GYCD84.2 32 | IgG1, κ | hD1 | 99.0% | 99.5% | 78.6% | 28.7% | 78.2% |
| GYCD84.3 89 | IgG1, κ | hD1 | 83.0% | 99.1% | 91.3% | 24.9% | 72.7% |
| GYCD84.1 97 | IgG2a, κ | hD1 | 97.7% | na | na | na | na |
| GYCD84.1 207 | IgG1, κ | hD1 | 94.2% | na | na | na | na |
| GYCD84.1 7 | IgG1, κ | hD2 | 92.6% | 91.4% | 59.0% | 19.0% | 64.4% |
| GYCD84.3 298 | IgG1, κ | hD2 | 93.5% | 45.9% | 28.3% | 16.6% | 53.9% |
| GYCD84.2 27 | IgG1, κ | hD2 | 96.3% | 97.5% | 66.3% | 25.4% | 77.5% |
| GYCD84.5 171 | IgG1, κ | hD2 | 93.2% | 73.9% | 35.1% | 26.8% | 64.8% |

The sequence corresponding to the variable region from the light chain (VL) and the sequence corresponding to the variable region from the heavy chain (VH) were determined from the different hybridomas using the Mouse Ig-Primer Set (Novagen). Sanger sequencing of this region was carried out by AbsoluteAntibody (United Kingdom). The antibody-sequence analysis was performed as follows: the complementarity-determining regions (CDR) and framework regions (FR) were identified using Abysis (sequences were defined by the Kabat-numbered scheme), IMGT and IgBLAST databases.

Table 5 shows the VH and VL sequence of these antibodies. The three CDRs in each sequence are highlighted in bold and underlined.

**Table 5. VH and VL sequences of anti-CD84 mouse monoclonal antibodies.**

| **Antibody** | **VH** | **VL** |
|---|---|---|
| 152-1D5 | QVQLQQPGAEVVQPGTSVKLSCKASGYNF TNYWINWVKLRPGQGLEWIGDIYPVSGTT NYNEKFKRKATLTVDTSSSTGNMQLSSLA SEDSALYYCASGTGRFAYWGQGTLVTVSA (SEQ ID NO: 61) | DIVLTQSPASLAVSLGQRATISCRASQSV STSSYSYMHWYQQKPGQPPKLLIKFASNL ESGVPARFSGSGSGTDFTLNIHPVEEEDT ATYYCQHSWEIPYTFGGGTKLEIK (SEQ ID NO: 62) |
| 153-4D9 | QVQLQQSGAELVRPGTSVKISCKASGYTF TNYWLGWVKQRPGHGLEWIGDIYPGGGYT NYIEKFKGKATLTADTSSSTAYMQLSSLT SEDSAVYFCARYEGGYYGNYDAMDYWGQG TSVTVSS (SEQ ID NO: 63) | DIVLTQSPGSLAVSLGQRATISCRASESV DNYGISFMNWFQQKPGQPPKLLIYAASNQ GSGVPARFSGSGSGTDFSLNIHPMEEDDT AMYFCQQSKAVPRTFGGGTKLEIK (SEQ ID NO: 64) |
| GYCD84.1-7 | QVTLKESGPGILQPSQTLSLTCSFSGFSL STSGMGVGWIRQPSGKGLEWLAHIWWDDV | DIQMTQSPASLSASVGETVTITCRASENI FSSLAWYQQRQGKSPQLLVYNAKTLAEGV |
| | KRYNPALKSRLTISKDTSSSQVFLKIASV DTADTATYYCARMRTSYYFDYWGQGTTLT VSS (SEQ ID NO: 74) | PSRFSGSGSGTQFSLKINSLQPEDFGSYY CQHHYATPFTFGSGTKLEIK (SEQ ID NO: 75) |
| GYCD84.1-97 | QVQLQQPGAELVKPGTSVKLSCKASGYNF TSYWINWVKLRPGQGLEWIGDIYLGSGST NYNEKFKSKATLTVDTSSSSAYMQLSSLV SEDSALYYCATSGGYLGYWGQGTTLTVSS (SEQ ID NO: 76) | DIVLTQSPASLAVSLGQRATISCRASQSV STSSYSYMHWYQQKPGQPPKLLIKFASNL ESGVPARFSGSGSGTDFTLNIHPVEEEDT ATYYCQHSWEIPYTFGGGTKLEIK (SEQ ID NO: 77) |
| GYCD84.1-108 | QVQLQQSGAELVRPGTSVKMSCKAAGYTF TNYWIGWVRQRPGHGLEWIGDIYPGGGYT NYNENFKGKATLTADTSSSTAYMQLSSLT SEDSAIYYCARSTTYYSSYWCFDVWGPGT TVTVSS (SEQ ID NO: 78) | DIVMTQSPSSLSVSAGEKVTMSCKSSQSL LNSGNQANYLAWYQQKPGQPPKLLINGAS TRESGVPDRFTGSGSGTDFTLTISSVQAE DLAVYYCQNDHSYPFTFGSGTKLEIK (SEQ ID NO: 79) |
| GYCD84.1-207 | EVKLLESGGGLVQPGGSLKLSCAASGFDF SRYWMSWVRQAPGKGLEWIGEINPDSSTI NYTPSLKDKFIISRDNAKNTLYLQMSKVR SEDTALYYCARPGPTVVATYWYFDVWGAG TTVTVSS (SEQ ID NO: 80) | DVLMTQTPLSLPVSLGDQASISCRSSQSI VHSNGNTYLEWYLQKPGQSPKLLIYKVSS RFSGVPDRFSGSASGTDFTLKISRVEAED LGVYYCFQGSHVPRTFGGGTKLEIK (SEQ ID NO: 81) |
| GYCD84.1-226 | QVQLQQPGAELVKPGTSVKLSCKASGYNF TRYWINWVKLRPGQGLEWIGDIYPGSGST NYNEKFKSKATLTVDTSSSTAYMQLSRLA SEDSALYYCARDTTIAYWGQGTLVTVSA (SEQ ID NO: 82) | DIVLTQSPTSLAVSLGQRATISCRASQSV TTSRYSYMHWYQQKPGQPPKLLIKFASNL ESGVPARFSGSGSGTDFTLNIHPVEEEDT ATYYCQHSWEIPYTFGGGTKLEIK (SEQ ID NO: 83) |
| GYCD84.2.32 | EVQLQQSGPELEKPGASVKISCKASGYSF TGYNMNWVKQSNGKSLEWIGNIDPYYGGT NYNQKFKGKATLTVDKSSSTAYMQLKSLT SEDSAVYYCARGLLSGSFPYWGQGTLVTV SA (SEQ ID NO: 161) | DIQMTQSPASLSASVGETVTITCRASENI YSYLAWYQQKQGKSPQLLVYNAKTLAEGV PSRFSGSGSGTQFSLKINSLHPEDFGSYF CQHHYGSPLTFGAGTKLEVK (SEQ ID NO: 162) |
| GYCD84.3.89 | EVKLLESGGGLVQPGGSLKLSCAASGFGF SRSWMSWVRQAPGKGLEWIGEINPDSSTI NYTPSLKDKFIISRDNAKNTLFLQMSKVR SEDTALYYCATFYDGYSIYWYFDVWGAGT TVTVSS (SEQ ID NO: 163) | DVLMTQTPLSLPVSLGDQASISCRSSQSI VHSNGDTYLEWYLQKPGQSPKLLIYKVSN RFSGVPDRFSGSGSGTDFTLKISRVEAED LGVYYCFQGSHVPRTFGGGTKLEIK (SEQ ID NO: 164) |
| GYCD84.3.298 | QVTLKESGPGILQPSQTLSLTCSFSGFSL STSGMGVGWIRQPSGKGLEWLAHIWWDDV KRYNPALRSRLTISKDTSSSQVFLNIASV DTADTATYYCTRIAVTYFFDFWGQGTTLT VSS (SEQ ID NO: 165) | DIQMTQSPASLSASVGETVTITCRASENI FSSFAWYQQKRGRSPQLLVYNARTLAEGV PSRFSGSGSGTQFSLKINSLQPEDFGTYF CQHHYASPFTFGSGTELGVK (SEQ ID NO: 166) |
| GYCD84.2.27 | QVTLKESGPGILQPSQTLSLTCSFSGFSL TTSGMGVGWIRQPSGKGLEWLAHIWWDDV KRYNPALKSRLTISKDTSSSQVFLKIASV DTADTASYYCARMSTSYYFDYWGQGTTLT VSS (SEQ ID NO: 167) | DIVMTQSHKFMSTSLGDRVSISCKASQSL FTSVAWYQQKPGQSPKLLIYSASYRYTGV PDRFTGSGSGTDFTFTISSVQAEDLAVYY CQQHYSSPFTFGSGTKLGIK (SEQ ID NO: 168) |
| GYCD84.5.171 | EVQLVESGGGLVQPKGSLKLACAASGFTF NIYAMNWVRQAPGKGLEWVARIRSKSNNY ARFYADSVKDRFTISRDDSQSMLYLQMNN LKTEDTAMYYCVRPLRSYFSMDYWGQGTS VTVSS (SEQ ID NO: 169) | SIVMTQSPKSMSMSVGERVTLSCKASENV DTYVSWYQQKPEQSPKLLIYGASNRYTGV PDRFTGSGSATDFTLTISSVKAEDLADYH CGQTYSYPWTFGGGTKLEIK (SEQ ID NO: 170) |

### Human scFvs

Phage display screening was used to identify novel human scFvs that bind to CD84. Three different scFvs were identified (R3-B3, R3-G7 and R3-H3); the sequence of the CDR and FR in the variable domains of the heavy (VH) and light (VL) chains is shown in Table 6.

**Table 6. VL and VH sequences of anti-CD84 human scFvs.**

| | **R3-B3** | **R3-G7** | **R3-H3** |
|---|---|---|---|
| **VH FR1** | QVQLVESGGGLVQPGGSLRL SCAAS (SEQ ID NO: 84) | QVQLVESGGGVVQPGRSLRL SCTAS (SEQ ID NO: 92) |  (SEQ ID NO: 100) |
| **VH CDR1** | GFTFSSYA (SEQ ID NO: 13) | GFTFSSYP (SEQ ID NO: 19) | GFTFSDNA (SEQ ID NO: 25) |
| **VH FR2** | MSWVRQAPGKGLEWVSA (SEQ ID NO: 85) | MHWVRQAPGKGLEWVAA (SEQ ID NO: 93) | MSWVRQAPGKGLEWVSA (SEQ ID NO: 101) |
| **VH CDR2** | ISGSGGST (SEQ ID NO: 14) | ISYHGRNK (SEQ ID NO: 20) | ISGTGRTT (SEQ ID NO: 26) |
| **VH FR3** | YYADSVKGRFTISRDNSKNT LYLQMNSLRAEDTAVYYC (SEQ ID NO: 86) | FYADSVKGRFTISRDDLRST LYLQMNNLKADDTAVYSC (SEQ ID NO: 94) | YYADSVKGRFTISRDNSKNT LYLQMNSLRAEDTAVYYC (SEQ ID NO: 102) |
| **VH CDR3** | AKWDCSDGRCYWAY (SEQ ID NO: 15) | ARDRDATPGGTGVGNHGMAV (SEQ ID NO: 21) | AKWDCSDGRCYWAY (SEQ ID NO: 27) |
| **VH FR4** | WGQGTLVTVSS (SEQ ID NO: 87) | WGQGTTVTVSS (SEQ ID NO: 95) | WGQGTLVTVSS (SEQ ID NO: 103) |
| **VH** | SEQ ID NO: 65 | SEQ ID NO: 68 | SEQ ID NO: 71 |
| **Linker** | GGGGSGGGGSGGGGSGGGGA S (SEQ ID NO: 67) | GGGGSGGGGSGGGGSGGGGA S (SEQ ID NO: 70) | GGGGSGGGGSGGGGSGGGGA S (SEQ ID NO: 73) |
| **VL FR1** | QPVLTQPPSVSVAPGQTARI SCGGD (SEQ ID NO: 88) | EIVLTQSPLSLPVTPGEPAS ISCKSS (SEQ ID NO: 96) | EIVLTQSPLSLPVTLGQPAS ISCRSS (SEQ ID NO: 104) |
| **VL CDR1** | NIESKD (SEQ ID NO: 16) | QSLLHSSGYNY (SEQ ID NO: 22) | QSLVYSDGDTY (SEQ ID NO: 28) |
| **VL FR2** | VHWYQRKSGQAPVLVVH (SEQ ID NO: 89) | LDWYLQKPGQSPQLLIH (SEQ ID NO: 97) | LNWFQQRPGQSPRRLIY (SEQ ID NO: 105) |
| **VL CDR2** | DDA (SEQ ID NO: 17) | MGS (SEQ ID NO: 23) | KVS (SEQ ID NO: 29) |
| **VL FR3** | DRPTGIPDRFSGSNSGNTAT LTISRVEAGDEADYYC (SEQ ID NO: 90) | NRASGVPARFSGSGSGTDFT LRISRVEAEDVGVYYC (SEQ ID NO: 98) | NRDSGVPDRFSGSGSGTDFT LKISRVEAEDVGVYYC (SEQ ID NO: 106) |
| **VL CDR3** | QVWDSSSDHVV (SEQ ID NO: 18) | MQGLQTPPT (SEQ ID NO: 24) | MQGTHWPPNT (SEQ ID NO: 30) |
| **VL FR4** | FGGGTQLTVL (SEQ ID NO: 91) | FGGGTKLEIK (SEQ ID NO: 99) | FGQGTRLEIK (SEQ ID NO: 107) |
| **VL** | SEQ ID NO: 66 | SEQ ID NO: 69 | SEQ ID NO: 72 |

### EXAMPLE 3: CD84 CAR engineering

We engineered several anti-CD84 CAR constructs (CAR84). The complete CAR84 sequence was cloned into the third-generation lentiviral vector pCCL (Dull et al. A Third-Generation Lentivirus Vector with a Conditional Packaging System. J. Virol. 1998:72:8463-8471), including the signal peptide, the scFv specific for CD84, the CD8a hinge and transmembrane regions, the co-stimulatory domain 4-1BB and the signalling domain CD3-zeta, under the control of an EF1α promoter (Figure 6).

Different versions of the scFv domain were designed based on the antibodies described above. These scFv differ in the order of VH and VL sequences (with some versions having only the VH chain) and in the linker used between the VH and VL, which uses either three (S3) or four (S4) motifs (Gly-Gly-Gly-Gly-Ser). The name attributed to each CAR version reflects the design of the scFv. The sequences of the different scFv domains synthesized and cloned in the pCCL vector are listed in Table 7.

**Table 7. Sequence and structure of the scFv domains used in each CD84 CAR designed.**

| **CAR name** | **Antibody/ scFv** | **1st domain** | **Linker** | **2nd domain** |
|---|---|---|---|---|
| **152.1** (VH) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) | | |
| **152.2** (VLS3VH) | 152-1D5 | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) | GGGGS GGGGS GGGGS (SEQ ID NO: 110) | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) |
| **152.3** (VLS4VH) | 152-1D5 | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) | GGGGS GGGGS GGGGS GGGGS (SEQ ID NO: 111) | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) |
| **152.4** (VHS3VL) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) | GGGGS GGGGS GGGGS (SEQ ID NO: 110) | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) |
| **152.5** (VHS4VL) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) | GGGGS GGGGS GGGGS GGGGS (SEQ ID NO: 111) | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) |
| **153.1** (VH) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | | |
| **153.2** (VLS3VH) | 153-4D9 | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) | GGGGS GGGGS GGGGS (SEQ ID NO: 110) | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) |
| **153.3** (VLS4VH) | 153-4D9 | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) | GGGGS GGGGS GGGGS GGGGS (SEQ ID NO: 111) | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) |
| **153.4** (VHS3VL) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | GGGGS GGGGS GGGGS (SEQ ID NO: 110) | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) |
| **153.5** (VHS4VL) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | GGGGS GGGGS GGGGS GGGGS (SEQ ID NO: 111) | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) |
| **B3.4** (VHS3VL) | R3-B3 | VH: QVQLVESGGGLVQPGGSLRLS CAASGFTFSSYAMSWVRQAPG KGLEWVSAISGSGGSTYYADS VKGRFTISRDNSKNTLYLQMN SLRAEDTAVYYCAKWDCSDGR CYWAYWGQGTLVTVSS (SEQ ID NO: 65) | GGGGS GGGGS GGGGS (SEQ ID NO: 110) | VL: QPVLTQPPSVSVAPGQTARIS CGGDNIESKDVHWYQRKSGQA PVLVVHDDADRPTGIPDRFSG SNSGNTATLTISRVEAGDEAD YYCQVWDSSSDHVVFGGGTQL TVL (SEQ ID NO: 66) |
| **B3.5** (VHS4VL) **G7.4** | R3-B3 R3-G7 | VH: QVQLVESGGGLVQPGGSLRLS CAASGFTFSSYAMSWVRQAPG KGLEWVSAISGSGGSTYYADS VKGRFTISRDNSKNTLYLQMN SLRAEDTAVYYCAKWDCSDGR CYWAYWGQGTLVTVSS (SEQ ID NO: 65) | GGGGS GGGGS GGGGS GGGGS (SEQ ID NO: 111) | VL: QPVLTQPPSVSVAPGQTARIS CGGDNIESKDVHWYQRKSGQA PVLVVHDDADRPTGIPDRFSG SNSGNTATLTISRVEAGDEAD YYCQVWDSSSDHVVFGGGTQL TVL (SEQ ID NO: 66) |
| (VHS3VL) | | VH: QVQLVESGGGVVQPGRSLRLS CTASGFTFSSYPMHWVRQAPG | GGGGS GGGGS GGGGS | VL: EIVLTQSPLSLPVTPGEPASI SCKSSQSLLHSSGYNYLDWYL |
| | | KGLEWVAAISYHGRNKFYADS VKGRFTISRDDLRSTLYLQMN NLKADDTAVYSCARDRDATPG GTGVGNHGMAVWGQGTTVTVS S (SEQ ID NO: 68) | (SEQ ID NO: 110) | QKPGQSPQLLIHMGSNRASGV PARFSGSGSGTDFTLRISRVE AEDVGVYYCMQGLQTPPTFGG GTKLEIK (SEQ ID NO: 69) |
| **G7.5** (VHS4VL) | R3-G7 | VH: QVQLVESGGGVVQPGRSLRLS CTASGFTFSSYPMHWVRQAPG KGLEWVAAISYHGRNKFYADS VKGRFTISRDDLRSTLYLQMN NLKADDTAVYSCARDRDATPG GTGVGNHGMAVWGQGTTVTVS S (SEQ ID NO: 68) | GGGGS GGGGS GGGGS GGGGS (SEQ ID NO: 111) | VL: EIVLTQSPLSLPVTPGEPASI SCKSSQSLLHSSGYNYLDWYL QKPGQSPQLLIHMGSNRASGV PARFSGSGSGTDFTLRISRVE AEDVGVYYCMQGLQTPPTFGG GTKLEIK (SEQ ID NO: 69) |
| **H3.4** (VHS3VL) | R3-H3 | VH: EVQLVESGGGLVQPGGSLRLS CAASGFTFSDNAMSWVRQAPG KGLEWVSAISGTGRTTYYADS VKGRFTISRDNSKNTLYLQMN SLRAEDTAVYYCAKWDCSDGR CYWAYWGQGTLVTVSS (SEQ ID NO: 71) | GGGGS GGGGS GGGGS (SEQ ID NO: 110) | VL: EIVLTQSPLSLPVTLGQPASI SCRSSQSLVYSDGDTYLNWFQ QRPGQSPRRLIYKVSNRDSGV PDRFSGSGSGTDFTLKISRVE AEDVGVYYCMQGTHWPPNTFG QGTRLEIK (SEQ ID NO: 72) |
| **H3.5** (VHS4VL) | R3-H3 | VH: EVQLVESGGGLVQPGGSLRLS CAASGFTFSDNAMSWVRQAPG KGLEWVSAISGTGRTTYYADS VKGRFTISRDNSKNTLYLQMN SLRAEDTAVYYCAKWDCSDGR CYWAYWGQGTLVTVSS (SEQ ID NO: 71) | GGGGS GGGGS GGGGS GGGGS (SEQ ID NO: 111) | VL: EIVLTQSPLSLPVTLGQPASI SCRSSQSLVYSDGDTYLNWFQ QRPGQSPRRLIYKVSNRDSGV PDRFSGSGSGTDFTLKISRVE AEDVGVYYCMQGTHWPPNTFG QGTRLEIK (SEQ ID NO: 72) |

Complete CAR sequences are shown in Table 8.

**Table 8. Complete CAR sequences.**

| **CAR name** | **Antibody/ scFv** | **CAR sequence** |
|---|---|---|
| **152.3** (VLS4VH) | 152-1D5 | MALPVTGLLLSLGLLLHAARPTGDIVLTQSPASLAVSLGQRATISCRASQSV STSSYSYMHWYQQKPGQPPKLLIKFASNLESGVPARFSGSGSGTDFTLNIHP VEEEDTATYYCQHSWEIPYTFGGGTKLEIKRAGGGGSGGGGSGGGGSGGGGS QVQLQQPGAEVVQPGTSVKLSCKASGYNFTNYWINWVKLRPGQGLEWIGDIY PVSGTTNYNEKFKRKATLTVDTSSSTGNMQLSSLASEDSALYYCASGTGRFA YWGQGTLVTVSATTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGK GHDGLYQGLSTATKDTYDALHMQALPPR (SEQ ID NO: 172) |
| **153.4** (VHS3VL) | 153-4D9 | MALPVTGLLLSLGLLLHAARPTGQVQLQQSGAELVRPGTSVKISCKASGYTF TNYWLGWVKQRPGHGLEWIGDIYPGGGYTNYIEKFKGKATLTADTSSSTAYM QLSSLTSEDSAVYFCARYEGGYYGNYDAMDYWGQGTSVTVSSGGGGSGGGGS GGGGSDIVLTQSPGSLAVSLGQRATISCRASESVDNYGISFMNWFQQKPGQP PKLLIYAASNQGSGVPARFSGSGSGTDFSLNIHPMEEDDTAMYFCQQSKAVP RTFGGGTKLEIKRATTTPAPRPPTPAPTIASQPLSLRPEACRPAAGGAVHTR GLDFACDIYIWAPLAGTCGVLLLSLVITLYCKRGRKKLLYIFKQPFMRPVQT TQEEDGCSCRFPEEEEGGCELRVKFSRSADAPAYQQGQNQLYNELNLGRREE YDVLDKRRGRDPEMGGKPQRRKNPQEGLYNELQKDKMAEAYSEIGMKGERRR GKGHDGLYQGLSTATKDTYDALHMQALPPR (SEQ ID NO: 173) |
| **153.5** (VHS4VL) | 153-4D9 | MALPVTGLLLSLGLLLHAARPTGQVQLQQSGAELVRPGTSVKISCKASGYTF TNYWLGWVKQRPGHGLEWIGDIYPGGGYTNYIEKFKGKATLTADTSSSTAYM QLSSLTSEDSAVYFCARYEGGYYGNYDAMDYWGQGTSVTVSSGGGGSGGGGS GGGGSGGGGSDIVLTQSPGSLAVSLGQRATISCRASESVDNYGISFMNWFQQ KPGQPPKLLIYAASNQGSGVPARFSGSGSGTDFSLNIHPMEEDDTAMYFCQQ SKAVPRTFGGGTKLEIKRATTTPAPRPPTPAPTIASQPLSLRPEACRPAAGG AVHTRGLDFACDIYIWAPLAGTCGVLLLSLVITLYCKRGRKKLLYIFKQPFM RPVQTTQEEDGCSCRFPEEEEGGCELRVKFSRSADAPAYQQGQNQLYNELNL GRREEYDVLDKRRGRDPEMGGKPQRRKNPQEGLYNELQKDKMAEAYSEIGMK GERRRGKGHDGLYQGLSTATKDTYDALHMQALPPR (SEQ ID NO: 174) |
| **G7.5** (VHS4VL) | R3-G7 | MALPVTGLLLSLGLLLHAARPTGQVQLVESGGGVVQPGRSLRLSCTASGFTF SSYPMHWVRQAPGKGLEWVAAISYHGRNKFYADSVKGRFTISRDDLRSTLYL QMNNLKADDTAVYSCARDRDATPGGTGVGNHGMAVWGQGTTVTVSSGGGGSG GGGSGGGGSGGGGSEIVLTQSPLSLPVTPGEPASISCKSSQSLLHSSGYNYL DWYLQKPGQSPQLLIHMGSNRASGVPARFSGSGSGTDFTLRISRVEAEDVGV YYCMQGLQTPPTFGGGTKLEIKTTTPAPRPPTPAPTIASQPLSLRPEACRPA AGGAVHTRGLDFACDIYIWAPLAGTCGVLLLSLVITLYCKRGRKKLLYIFKQ PFMRPVQTTQEEDGCSCRFPEEEEGGCELRVKFSRSADAPAYQQGQNQLYNE LNLGRREEYDVLDKRRGRDPEMGGKPQRRKNPQEGLYNELQKDKMAEAYSEI GMKGERRRGKGHDGLYQGLSTATKDTYDALHMQALPPR (SEQ ID NO: 175) |
| **H3.5** (VHS4VL) | R3-H3 | MALPVTGLLLSLGLLLHAARPTGEVQLVESGGGLVQPGGSLRLSCAASGFTF SDNAMSWVRQAPGKGLEWVSAISGTGRTTYYADSVKGRFTISRDNSKNTLYL QMNSLRAEDTAVYYCAKWDCSDGRCYWAYWGQGTLVTVSSGGGGSGGGGSGG GGSEIVLTQSPLSLPVTLGQPASISCRSSQSLVYSDGDTYLNWFQQRPGQSP RRLIYKVSNRDSGVPDRFSGSGSGTDFTLKISRVEAEDVGVYYCMQGTHWPP NTFGQGTRLEIKTTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGK GHDGLYQGLSTATKDTYDALHMQALPPR (SEQ ID NO: 176) |
| **1.7.3** (VLS4VH) | GYCD84. 1-7 | MALPVTGLLLSLGLLLHAARPTGDIQMTQSPASLSASVGETVTITCRASENI FSSLAWYQQRQGKSPQLLVYNAKTLAEGVPSRFSGSGSGTQFSLKINSLQPE DFGSYYCQHHYATPFTFGSGTKLEIKGGGGSGGGGSGGGGSGGGGSQVTLKE SGPGILQPSQTLSLTCSFSGFSLSTSGMGVGWIRQPSGKGLEWLAHIWWDDV KRYNPALKSRLTISKDTSSSQVFLKIASVDTADTATYYCARMRTSYYFDYWG QGTTLTVSSTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD KRRGRDPEMGGKPQRRKNPQEGLYNELQKDKMAEAYSEIGMKGERRRGKGHD GLYQGLSTATKDTYDALHMQALPPR (SEQ ID NO: 177) |
| **1.7.5** (VHS4VL) | GYCD84. 1-7 | MALPVTGLLLSLGLLLHAARPTGQVTLKESGPGILQPSQTLSLTCSFSGFSL STSGMGVGWIRQPSGKGLEWLAHIWWDDVKRYNPALKSRLTISKDTSSSQVF LKIASVDTADTATYYCARMRTSYYFDYWGQGTTLTVSSGGGGSGGGGSGGGG SGGGGSDIQMTQSPASLSASVGETVTITCRASENIFSSLAWYQQRQGKSPQL LVYNAKTLAEGVPSRFSGSGSGTQFSLKINSLQPEDFGSYYCQHHYATPFTF GSGTKLEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD KRRGRDPEMGGKPQRRKNPQEGLYNELQKDKMAEAYSEIGMKGERRRGKGHD GLYQGLSTATKDTYDALHMQALPPR (SEQ ID NO: 178) |
| **1.226.5** (VHS4VL) | GYCD84. 1-226 | MALPVTGLLLSLGLLLHAARPTGQVQLQQPGAELVKPGTSVKLSCKASGYNF TRYWINWVKLRPGQGLEWIGDIYPGSGSTNYNEKFKSKATLTVDTSSSTAYM QLSRLASEDSALYYCARDTTIAYWGQGTLVTVSAGGGGSGGGGSGGGGSGGG GSDIVLTQSPTSLAVSLGQRATISCRASQSVTTSRYSYMHWYQQKPGQPPKL LIKFASNLESGVPARFSGSGSGTDFTLNIHPVEEEDTATYYCQHSWEIPYTF GGGTKLEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD |
| | | KRRGRDPEMGGKPQRRKNPQEGLYNELQKDKMAEAYSEIGMKGERRRGKGHD GLYQGLSTATKDTYDALHMQALPPR (SEQ ID NO: 179) |
| **3.89.5** (VHS4VL) | GYCD84. 3-89 | MALPVTGLLLSLGLLLHAARPTGEVKLLESGGGLVQPGGSLKLSCAASGFGF SRSWMSWVRQAPGKGLEWIGEINPDSSTINYTPSLKDKFIISRDNAKNTLFL QMSKVRSEDTALYYCATFYDGYSIYWYFDVWGAGTTVTVSSGGGGSGGGGSG GGGSGGGGSDVLMTQTPLSLPVSLGDQASISCRSSQSIVHSNGDTYLEWYLQ KPGQSPKLLIYKVSNRFSGVPDRFSGSGSGTDFTLKISRVEAEDLGVYYCFQ GSHVPRTFGGGTKLEIKTTTPAPRPPTPAPTIASQPLSLRPEACRPAAGGAV HTRGLDFACDIYIWAPLAGTCGVLLLSLVITLYCKRGRKKLLYIFKQPFMRP VQTTQEEDGCSCRFPEEEEGGCELRVKFSRSADAPAYQQGQNQLYNELNLGR REEYDVLDKRRGRDPEMGGKPQRRKNPQEGLYNELQKDKMAEAYSEIGMKGE RRRGKGHDGLYQGLSTATKDTYDALHMQALPPR (SEQ ID NO: 180) |

### CD84 CART production

Lentiviruses (LV) containing the different versions of the pCCL-EF1α-CD84 vectors were produced in HEK293-T cells and the number of transducing units was determined by the limiting dilution method. The lentiviruses were then used to transduce T cells isolated from whole blood. These CAR-T cells were then expanded for 6 to 8 days.

The following tables (Tables 9-11) summarize the number of T cells obtained after three different transductions for the different CARTs, as well as the percentage of these T cells that expressed the CD84 CAR on their surface.

**Table 9. Number and percentage of CAR-positive T cells obtained following three independent transductions with lentivirus expressing CD84 CARs based on the 152-1D5 antibody.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 19.2 ×10⁶ | 0% | 8.7 ×10⁶ | 0.0% | 7.8 ×10⁶ | 0.0% |
| **152.1** | 4.6 ×10⁶ | 2.5% | 5.5 ×10⁶ | 2.1% | 6.2 ×10⁶ | 2.3% |
| **152.2** | 0.9 ×10⁶ | 66.0% | 1.0 ×10⁶ | 87.8% | 1.0 ×10⁶ | 86.3% |
| **152.3** | 2.1 ×10⁶ | 66.3% | 0.5 ×10⁶ | 83.9% | 0.7 ×10⁶ | 89.1% |
| **152.4** | 1.6 ×10⁶ | 38.2% | 2.0 ×10⁶ | 60.7% | 1.5 ×10⁶ | 69.5% |
| **152.5** | 1.1 ×10⁶ | 47.7% | 2.6 ×10⁶ | 75.4% | 2.4 ×10⁶ | 73.8% |

**Table 10. Number and percentage of CAR-positive T cells obtained following three independent transductions with lentivirus expressing CD84 CARs based on the 153-4D9 antibody.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 5.7 ×10⁶ | 0% | 18.4 ×10⁶ | 0% | 12.3 ×10⁶ | 0% |
| **153.1** | 0.2 ×10⁶ | 0% | 13.9 ×10⁶ | 1.4% | 11.6 ×10⁶ | 0.5% |
| **153.2** | 5.2 ×10⁶ | 1.3% | 13.0 ×10⁶ | 4.5% | 8.3 ×10⁶ | 0.5% |
| **153.3** | 6.5 ×10⁶ | 19.4% | 11.3 ×10⁶ | 43.4% | 7.3 ×10⁶ | 60.5% |
| **153.4** | 5.7 ×10⁶ | 39.2% | 7.6 ×10⁶ | 39.8% | 5.5 ×10⁶ | 34.7% |
| **153.5** | 4.3 ×10⁶ | 52.1% | 12.6 ×10⁶ | 51.7% | 4.5 ×10⁶ | 58.7% |

**Table 11. Number and percentage of CAR-positive T cells obtained following three independent transductions with lentivirus expressing CD84 CARs based on the R3-B3, R3-G7 and R3-H3 scFvs.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 41.8 ×10⁶ | 0% | 79.4 ×10⁶ | 0% | 27.3 ×10⁶ | 0% |
| **B3.4** | 55.4 ×10⁶ | 20.4% | 49.1 ×10⁶ | 6.5% | 22.3 ×10⁶ | 7.8% |
| **B3.5** | 47.5 ×10⁶ | 11.6% | 38.9 ×10⁶ | 3.8% | 16.6 ×10⁶ | 5.3% |
| **G7.4** | 49.9 ×10⁶ | 56.3% | 43.4 ×10⁶ | 30.7% | 32.6 ×10⁶ | 39.1% |
| **G7.5** | 42 ×10⁶ | 64.3% | 48.8 ×10⁶ | 34% | 10.3 ×10⁶ | 47.4% |
| **H3.4** | 18.7 ×10⁶ | 51.9% | 59.9 ×10⁶ | 54% | 19.4 ×10⁶ | 33.7% |
| **H3.5** | 10.3 ×10⁶ | 55.6% | 52.5 ×10⁶ | 62.7% | 34.6 ×10⁶ | 40.2% |

The following CAR LVs did not lead to an efficient expansion of CAR-positive T cells: 152.1, 153.1, 153.2, B3.4 and B3.5. The 152.1 and 153.1 CARs were not used in further experiments.

### EXAMPLE 4: CD84 CART in vitro cytokine production

To assess the ability of each CART cell to release cytokines, the supernatant of the effector-target cell co-cultures were collected. The CD84^{high} cell line Ramos was used as the target cell at a 2:1 effector:target cell ratio. The levels of IFN-γ (Figure 7), IL-2 (Figure 8), granzyme-B (Figure 9) and TNF-α (Figure10) in the supernatants was determined by enzyme linked immunosorbent assays (ELISA) after 24 hours of co-culture. Untransduced T cells (UT) were used as negative control (in co-culture with the target cells). Four different independent experiments were performed.

These results showed that CART cells with the R3-B3 scFv CAR binding domain lack cytokine release activity and therefore were not used in subsequent experiments. On the other hand, CART cells with the R3-H3 scFv CAR binding domain released a high amount of cytokines, with these two CART cells (H3.4 and H3.5) being the ones with the largest proinflammatory profile (based on TNF-α secretion), followed by CART cells with the 152-1D5 antibody CAR binding domain, which also show a high cytokine release profile. The 152-1D5 CART cells showed a similar profile independently of the scFv design (VH-VL order and linker length). CART cells based on the 153-4D9 and R3-G7 antibodies have a similar profile, with lower cytokine release in comparison to R3-H3 and 152-1D5 CART cells, except for 153.3 CART cells, which barely secrete cytokines.

### EXAMPLE 5: Anti-CD84 CART in vitro cytotoxicity

To evaluate the efficacy of the CART84 cells *in vitro,* we performed cytotoxicity assays of each CART cell against several GFP-expressing target cell lines with different levels of CD84 expression and from both lymphoid (Ramos, NALM6 and MOLT-4 cell lines) and myeloid origin (K562 and Kasumi-1 cell lines). CART cytotoxicity was assessed after a 24-hour co-culture by determining the percentage of live GFP-positive cells by cytometry. Effector:target cell ratios of 4:1, 2:1. 1:1 and 0.5:1 were tested.

First, we tested all the CARs against the CD84^{high} Ramos cell line (Figure 11). The results for the effector:target ratio of 2:1 are shown. R3-H3 and 152-1D5 CART cells showed the highest cytotoxic activity against this cell line, while 153-4D9 and R3-G7 CART cells showed a lower cytotoxic activity. 153.3 CART cells showed the lowest cytotoxic activity of all CARs based on 153-4D9 constructs.

Next, we tested all the CARs against K562, a myeloid cell line with a low expression of CD84 (Figure 12). The results for the effector:target ratio of 2:1 are shown. CARs based on the 152-1D5 and 153-4D9 antibodies showed a statistically significant cytotoxic activity in comparison with UT cells.

Based on the cytotoxicity against Ramos and K562, we selected the following CART cells for additional characterization: 152.3, 153.4, 153.5, G7.5 and H3.5. We assessed the cytotoxicity of these CART cells at four effector:target ratios (4:1, 2:1, 1:1 and 0.5:1) against several cell lines after 24 and 48 hours of co-culture. Ramos is an aggressive B-cell lymphoma cell line (Figure 13), K562 is an acute myeloid leukaemia cell line with low CD84 expression (Figure 14) whereas Kasumi-1 is an acute myeloid leukaemia cell line with moderate CD84 expression (Figure 15). NALM-6 is a B-cell acute lymphoblastic leukaemia cell line (Figure 16) and MOLT-4 a T-cell acute lymphoblastic leukaemia cell line (Figure 17).

As shown for the 2:1 ratio (Figure 11), the selected CARs displayed a statistically significant cytotoxic activity against Ramos cells for each of the effector:target ratios in comparison with UT cells (Figure 13). In the case of K562, the same pattern shown in Figure 12 was observed, with CARs based on the 152-1D5 and 153-4D9 antibodies showing a statistically significant cytotoxic activity in comparison with UT cells (Figure 14).

All the selected CARs showed cytotoxic activity against Kasumi-1, with CARs based on 152.1D5 and 153.4D9 with a higher cytotoxic effect than those based on R3-G7 and R3-H3 antibodies (Figure 15).

CARs based on 152.1D5 and 153.4D9 antibodies showed a higher cytotoxic activity against NALM-6 cell line than CARs based on R3-G7 and R3-H3 scFv (Figure 16). For the MOLT-4 cell line, the CAR based on the 152-1D5 antibody showed the highest statistically significant cytotoxic activity, compared to UT cells, followed by CARs based on 153-4D9 and R3-G7 antibodies (Figure 17).

### EXAMPLE 6: CD84 expression in peripheral blood mononuclear cells (PBMC)

We assessed CD84 expression in the different cell populations of PBMCs by flow cytometry, and obtained results similar to what has been previously described. Monocytes show high CD84 expression, similar to that observed in the Ramos cell line. B cells display moderate CD84 expression. Both CD4 and CD8 T cells have two distinct subpopulations with regards to CD84 expression (positive and negative), with moderate CD84 expression levels on the positive population (Figure 18).

### EXAMPLE 7: CD84 CART in vitro cytotoxicity against PBMCs

We assessed the cytotoxic activity of CD84 CART against PBMCs. For each experiment, both effector cells (i.e. the CART84 cells) and the target cells (PBMCs) were obtained from the same donor. The mean of three independent experiments of three different donors are shown. CART cells did not display statistically significant cytotoxic activity against their own PBMCs. However, these CART cells did show statistically significant cytotoxic activity against Ramos cells in a parallel experiment (Figure 19).

### EXAMPLE 8: Methods

### Generation of mouse monoclonal anti-CD84 antibodies

BALB/c mice were immunized three to four times, at 3-week intervals, with 300.19 cells stably transfected with the CD84 full-length DNA (de la Fuente MA et al. CD84 leukocyte antigen is a new member of the Ig superfamily. Blood. 1997; 15;90(6):2398-405). The first intraperitoneal (i.p.) injection consisted of 20×10⁶ cells in 300 µl PBS, the second consisted of 20×10⁶ cells in 300 µl of PBS and the final injection consisted of 30×10⁶ cells in 300 µl in PBS. Mice were euthanized on the third day after the final boost and the splenocytes were harvested to perform the cell fusion.

NS1 myeloma cells (European Collection of Cell Cultures, Salisbury, UK) were fused with spleen cells by incubating them at a ratio of 4:1 (splenocytes:NS1) at 37°C, centrifuging for 10 minutes at RT and adding 1 mL of warm PEG solution to the cell pellet while constantly swirling. Cells were slowly resuspended in RPMI culture medium, centrifuged and incubated at 37°C in 5% CO₂ in a humidified incubator.

Screening was carried out 10 days after fusion by analysing 50µl of hybridoma supernatant by flow cytometry with 300.19-CD84 cells, using untransfected 300.19 cells as a negative control. Hybridomas that tested positive to 300.19-CD84 and negative to 300.19 cells were transferred to 24-well plates, grown until confluent and then transferred to T75 flasks. Single hybridoma clones producing antibodies of interest were then isolated by limited dilution. For each hybridoma, 10 single clones were retested by flow cytometry with 300.19-CD84 cells. The cloning protocol was repeated with one of the positive clones.

For each antibody, isotype (class and subclass) was determined by ELISA using anti-IgG coated plates to which the antibodies were added and then incubating the plates with an anti-mouse HRP antibody. To determine which of the two CD84 extracellular domains is recognized by the antibody, flow cytometry analysis was carried out with COS cells expressing a chimeric CD84 extracellular domain in which the domain 1 (D1) and 2 (D2) sequence is either human or murine.

### Identification of fully human anti-CD84 scFv

Human CD84 produced by mammalian cells (checked by SDS-PAGE; Figure 20) was used as an antigen to carry out panning of a human naïve phage display LiAb SFMax library (Proteogenix, France) with high diversity of 5.37×10¹⁰ scFv variants.

For the biopanning rounds, tubes were coated with antigen, blocked, washed and incubated with the phage library. After washing, elution of phage binders was done with Glycine HCI followed by neutralization.

To determine the concentration of the eluted phages, these were added to *E. coli* TG1 cells which were then poured onto plates and cultured upside down. The calculation of PFU (plaque-forming units) was based on the number of plaques (i.e. dead TG1) on a plate.

To amplify the eluted phages, these were added to *E. coli* TG1 cells which were then infected by helper phage and culture. Phage precipitation was carried out with PEG/NaCl followed by resuspension and the amplified phages used in the next biopanning round of ELISA analysis.

This analysis showed significant enrichment over the rounds (Table 12) and since round 3 output already showed excellent enrichment, no additional biopanning rounds to prevent decrease in phage diversity.

**Table 12. Concentration of eluted phages**

| **Rounds** | **Antigen concentration** | **Number of washings** | **Phage quantity (pfu)** | |
|---|---|---|---|---|
| | | | **Input** | **Output** |
| 1 | 50 µg/mL | 5 | 2×10¹² | 2.5×10⁵ |
| 2 | 50 µg/mL | 5 | 2×10¹² | 4.5×10⁶ |
| 3 | 50 µg/mL | 5 | 2×10¹² | 9×10⁷ |

Phages from round 3 were selected for monoclonal ELISA analysis. Single *E. coli* TG1 clones were picked and cultured with helper phage. Following centrifugation, supernatants containing phages were collected.

Plates were coated with either antigen or buffer, washed, blocked and washed again. Phages were then added to plates and incubated. Plates were washed and then an anti-phage horseradish peroxidase (HRP) antibody was added, followed by washing and incubation of TMB followed by HCl. Plates were read at 450 nm.

After sequencing of positive clones, 3 different unique sequences were identified. The 3 unique clones identified were then re-tested by ELISA to ensure clone specificity. All phages were tested at the same concentration. Plates were either covered with antigen (Ag) or with buffer (NC). The results clearly confirmed that the 3 clones specifically bind to the CD84 antigen (Table 13).

**Table 13. ELISA of positive clones**

| **Clone ID** | **Ag¹** | **NC²** |
|---|---|---|
| R3-B3 | 0.5060 | 0.0240 |
| R3-G7 | 2.397 | 0.0220 |
| R3-H3 | 0.696 | 0.0170 |

### Donors, cell lines

Healthy donor blood buffy-coats were obtained from the reference blood bank Banc de Sang i Teixits, Barcelona (Spain).

Ramos, Raji, NALM6, K562, Kasumi-1, Kasumi-6, THP-1, and U937 were purchased from the American Type Culture Collection (ATCC). Ramos, Raji, NALM6, K562, NS1 and THP-1 cell lines were cultured in RPMI medium (ThermoFisher) supplemented with 10% foetal bovine serum (FBS, Merck) and penicillin-streptomycin (Labclinics), 300.19 cells were supplemented also with 1% L-glutamine (Gibco) and 0.1% 2-Beta-Mercaptoethanol (Sigma). The HEK293T and the COS cell line was cultured in DMEM medium (Gibco) supplemented with 10% FBS (Merck) and penicillin-streptomycin (Labclinics) and with 1% L-glutamine (Gibco). All cell lines were grown at 37°C and 5% CO₂.

### Lentivirus production

HEK293-T cells were transfected with our transfer vector pCCL-EF1α-CD84 together with the packaging plasmids pRSV-Rev (Addgene, 12253), pMDLg/Prre (Addgene, 12251), and envelope plasmid pCMV-VSV-G (Addgene, 12259) using linear polyethylenimine (PEI, molecular weight 25000, Polysciences Inc 23966-1). Lentiviral supernatant was collected 48 hours later and concentrated with LentiX-Concentrator (Clontech, Takara) following the manufacturer's protocol. Concentrated lentivirus was stored at -80°C until use.

### Lentivirus titration

The number of transducing units (TU/mL) was determined by the limiting dilution method.

HEK293T cells were seeded 24 h before transduction and 1:10 dilutions of the viral supernatant were prepared and added in DMEM medium (Gibco) supplemented with polybrene (Sigma-Aldrich) at 8 mg/mL. Cells were trypsinyzed 48 h later and labelled with AffiniPure F(ab')2 fragment goat anti-mouse immunoglobulin G (IgG) allophycocyanin (APC)-conjugated (Jacksonlmmuno Research Laboratories, 115-136-072). A dilution corresponding to 2%-20% of positive cells was used to calculate viral titer.

### T-cell transduction and CART expansion

T cells were isolated from whole blood during density gradient centrifugation with Ficoll-Paque^{™} by RosetteSep^{™} (RosetteSep Human T cell Enrichment cocktail from StemCell). T cells were cultured in X-Vivo 15 Serum Free Cell Medium (Lonza) supplemented with 5% AB human serum (Sigma H4522), penicillin-streptomycin (ThermoFisher, 100 mg/mL), and IL-2 at 50 IU/mL (Miltenyi). Cells were then activated using beads conjugated with CD3 and CD28 mAbs (Dynabeads Human T-Activator CD3/CD28 Gibco, 11131D). Twenty-four hours later they were transduced with the lentivirus in the presence of polybrene (Sigma-Aldrich) at 8 ug/mL. An expansion of 6 to 8 days was required before conducting the experiments.

### Flow cytometry

CAR84 was detected with a recombinant CD84-His protein (R&D, 1855-CD) and a secondary His Tag APC-conjugated antibody (R&D, IC050A) and with a biotinSP-conjugated AffiniPure F(ab')2-fragment goat-anti-mouse IgG (Jackson ImmunoResearch Laboratories, 115-065-072) or with a biotin SP-conjugated AffiniPure F(ab')2-fragment goat-anti-human IgG (Jackson ImmunoResearch Laboratories, 109-065-006) with a BV421-conjugated streptavidin (BD Horizon, 563259). The following mAbs against human proteins were used: CD3-APC, CD4-PE-Cy7, CD4-Alexa Fluor-488, CD19-PE, CD8-APC-H7, PD-1- PE-Cy7, TIM-3- BB515, CD69-PerCP-Cy^{™}5.5, LAG-3-BV-605, CD62L-FITC, CCR7-PerCP-Cy^{™}5.5, CD84-PE, CD4-PE-Cy^{™}7, CD45RA-APC (Becton Dickinson), CD84-APC and CD84-PE (BioLegend). Samples were run through the flow cytometer BD FACSCanto II (BD Biosciences), LSR II Fortessa 4L with HTS (BD), and Attune NxT 4L cytometer (ThermoFisher). For ELISA the following mAbs were used: an anti-mouse IgG-HRP (anti-mouse IgG-peroxidase antibody produced in goat, Sigma, cat: A3673-1ML) and an anti-mouse IgG coating (anti-mouse IgG antibody produced in goat (sigma, cat: M2650-1ML) Data were analysed using the FlowJo Software 10.7.1.

### In vitro cytotoxicity assays

The cytotoxicity of CART cells was assessed using different effector:target ratios and at different time-points. Target cells used in these assays were modified with a lentiviral vector to over-express GFP-firefly luciferase (GFP-ffLuc) as previously described (Shah et al. Antigen Presenting Cell-Mediated Expansion of Human Umbilical Cord Blood Yields Log-Scale Expansion of Natural Killer Cells with Anti-Myeloma Activity. PLoS One. 2013; 8(10)).

The percentage of remaining alive GFP⁺ tumour cells was analysed by flow cytometry and calculated using the following formula: % of live cell = 100 × (number of GFP⁺ cells with T cells at time x / number of GFP⁺ cells alone at time x).

### In vitro proliferation assays

CART84 cell proliferation in response to CD84 antigen was measured using a CFSE assay (Castella, M. et al. (2019) Mol. Ther. - Methods Clin. Dev. 12: 134-144). CART cells were stained with CellTrace CFSE (Invitrogen, ThermoFisher, 15598431) before being co-cultured with or without stimuli for 96 hours. Proliferation was analyzed by flow cytometry and proliferating index (PI) was calculated (PI = sum of number of cells in different generations / number of cells).

### In vitro cytokine production

IFN-γ, TNF-α, IL-6, IL-1β cytokines were quantified by Enzyme-Linked ImmunoSorbent Assay (DuoSet ELISA, R&D systems) following manufacturer's protocol.

All publications mentioned in the above specification are herein incorporated by reference. Various modifications and variations of the disclosed antigen-binding domains, antibodies, chimeric antigen receptors, uses and methods of the invention will be apparent to the skilled person without departing from the scope and spirit of the invention. Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person, are intended to be within the scope of the following claims.

## Claims

1. An antigen-binding domain comprising:
a) a heavy chain variable region (VH) having complementarity determining regions (CDRs) with the sequences: CDR1 - NYWIN (SEQ ID NO: 1); CDR2 - DIYPVSGTTNYNEKFKR (SEQ ID NO: 2); and CDR3 - GTGRFAY (SEQ ID NO: 3); or variants thereof each having up to three amino acid substitutions, additions or deletions; and
a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6); or variants thereof each having up to three amino acid substitutions, additions or deletions;
b) a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9), or variants thereof each having up to three amino acid substitutions, additions or deletions; and
a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12), or variants thereof each having up to three amino acid substitutions, additions or deletions;
c) a heavy chain variable region (VH) having complementarity determining regions (CDRs) with the sequences: CDR1 -GFTFSSYA (SEQ ID NO: 13); CDR2 - ISGSGGST (SEQ ID NO: 14); and CDR3 - AKWDCSDGRCYWAY (SEQ ID NO: 15), or variants thereof each having up to three amino acid substitutions, additions or deletions; and
a light chain variable region (VL) having CDRs with the sequences: CDR1 - NIESKD (SEQ ID NO: 16); CDR2 - DDA (SEQ ID NO: 17); and CDR3 - QVWDSSSDHVV (SEQ ID NO: 18), or variants thereof each having up to three amino acid substitutions, additions or deletions;
d) a heavy chain variable region (VH) having complementarity determining regions (CDRs) with the sequences: CDR1 -GFTFSSYP (SEQ ID NO: 19); CDR2 - ISYHGRNK (SEQ ID NO: 20); and CDR3 - ARDRDATPGGTGVGNHGMAV (SEQ ID NO: 21), or variants thereof each having up to three amino acid substitutions, additions or deletions; and
a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24), or variants thereof each having up to three amino acid substitutions, additions or deletions;
e) a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3-AKWDCSDGRCYWAY (SEQ ID NO: 27), or variants thereof each having up to three amino acid substitutions, additions or deletions; and
a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30), or variants thereof each having up to three amino acid substitutions, additions or deletions;
f) a heavy chain variable region (VH) having complementarity determining regions (CDRs) with the sequences: CDR1 -TSGMGVG (SEQ ID NO: 31); CDR2 - HIWWDDVKRYNPALKS (SEQ ID NO: 32); and CDR3 - MRTSYYFDY (SEQ ID NO: 33), or variants thereof each having up to three amino acid substitutions, additions or deletions; and
a light chain variable region (VL) having CDRs with the sequences: CDR1 - RASENIFSSLA (SEQ ID NO: 34); CDR2 - NAKTLAE (SEQ ID NO: 35); and CDR3 - QHHYATPFT (SEQ ID NO: 36), or variants thereof each having up to three amino acid substitutions, additions or deletions;
g) a heavy chain variable region (VH) having complementarity determining regions (CDRs) with the sequences: CDR1 - SYWIN (SEQ ID NO: 37); CDR2 - DIYLGSGSTNYNEKFKS (SEQ ID NO: 38); and CDR3 - SGGYLGY (SEQ ID NO: 39), or variants thereof each having up to three amino acid substitutions, additions or deletions; and
a light chain variable region (VL) having CDRs with the sequences: CDR1 - RASQSVSTSSYSYMH (SEQ ID NO: 40); CDR2 - FASNLES (SEQ ID NO: 41); and CDR3 - QHSWEIPYT (SEQ ID NO: 42), or variants thereof each having up to three amino acid substitutions, additions or deletions;
h) a heavy chain variable region (VH) having complementarity determining regions (CDRs) with the sequences: CDR1 - NYWIG (SEQ ID NO: 43); CDR2 - DIYPGGGYTNYNENFKG (SEQ ID NO: 44); and CDR3 - STTYYSSYWCFDV (SEQ ID NO: 45), or variants thereof each having up to three amino acid substitutions, additions or deletions; and
a light chain variable region (VL) having CDRs with the sequences: CDR1 - KSSQSLLNSGNQANYLA (SEQ ID NO: 46); CDR2 - GASTRES (SEQ ID NO: 47); and CDR3 - QNDHSYPFT (SEQ ID NO: 48), or variants thereof each having up to three amino acid substitutions, additions or deletions;
i) a heavy chain variable region (VH) having complementarity determining regions (CDRs) with the sequences: CDR1 - RYWMS (SEQ ID NO: 49); CDR2 - EINPDSSTINYTPSLKD (SEQ ID NO: 50); and CDR3 - PGPTVVATYWYFDV (SEQ ID NO: 51), or variants thereof each having up to three amino acid substitutions, additions or deletions; and
a light chain variable region (VL) having CDRs with the sequences: CDR1 - RSSQSIVHSNGNTYLE (SEQ ID NO: 52); CDR2 - KVSSRFS (SEQ ID NO: 53); and CDR3 - FQGSHVPRT (SEQ ID NO: 54), or variants thereof each having up to three amino acid substitutions, additions or deletions; or
j) a heavy chain variable region (VH) having complementarity determining regions (CDRs) with the sequences: CDR1 - RYWIN (SEQ ID NO: 55); CDR2 - DIYPGSGSTNYNEKFKS (SEQ ID NO: 56); and CDR3 - DTTIAY (SEQ ID NO: 57), or variants thereof each having up to three amino acid substitutions, additions or deletions; and
a light chain variable region (VL) having CDRs with the sequences: CDR1 - RASQSVTTSRYSYMH (SEQ ID NO: 58); CDR2 - FASNLES (SEQ ID NO: 59); and CDR3 - QHSWEIPYT (SEQ ID NO: 60), or variants thereof each having up to three amino acid substitutions, additions or deletions;
k) a heavy chain variable region (VH) having complementarity determining regions (CDRs) with the sequences: CDR1 - GYNMN (SEQ ID NO: 131); CDR2 - NIDPYYGGTNYNQKFKG (SEQ ID NO: 132); and CDR3 - GLLSGSFPY (SEQ ID NO: 133), or variants thereof each having up to three amino acid substitutions, additions or deletions; and
a light chain variable region (VL) having CDRs with the sequences: CDR1 - RASENIYSYLA (SEQ ID NO: 134); CDR2 - NAKTLAE (SEQ ID NO: 135); and CDR3 - QHHYGSPLT (SEQ ID NO: 136), or variants thereof each having up to three amino acid substitutions, additions or deletions;
l) a heavy chain variable region (VH) having complementarity determining regions (CDRs) with the sequences: CDR1 - RSWMS (SEQ ID NO: 137); CDR2 - EINPDSSTINYTPSLKD (SEQ ID NO: 138); and CDR3 - FYDGYSIYWYFDV (SEQ ID NO: 139), or variants thereof each having up to three amino acid substitutions, additions or deletions; and
a light chain variable region (VL) having CDRs with the sequences: CDR1 - RSSQSIVHSNGDTYLE (SEQ ID NO: 140); CDR2 - KVSNRFS (SEQ ID NO: 141); and CDR3 - FQGSHVPRT (SEQ ID NO: 142), or variants thereof each having up to three amino acid substitutions, additions or deletions;
m) a heavy chain variable region (VH) having complementarity determining regions (CDRs) with the sequences: CDR1 - TSGMGVG (SEQ ID NO: 143); CDR2 - HIWWDDVKRYNPALRS (SEQ ID NO: 144); and CDR3 - IAVTYFFDF (SEQ ID NO: 145), or variants thereof each having up to three amino acid substitutions, additions or deletions; and
a light chain variable region (VL) having CDRs with the sequences: CDR1 - RASENIFSSFA (SEQ ID NO: 146); CDR2 - NARTLAE (SEQ ID NO: 147); and CDR3 - QHHYASPFT (SEQ ID NO: 148), or variants thereof each having up to three amino acid substitutions, additions or deletions;
n) a heavy chain variable region (VH) having complementarity determining regions (CDRs) with the sequences: CDR1 - TSGMGVG (SEQ ID NO: 149); CDR2 - HIWWDDVKRYNPALKS (SEQ ID NO: 150); and CDR3 - MSTSYYFDY (SEQ ID NO: 151), or variants thereof each having up to three amino acid substitutions, additions or deletions; and
a light chain variable region (VL) having CDRs with the sequences: CDR1 - KASQSLFTSVA (SEQ ID NO: 152); CDR2 - SASYRYT (SEQ ID NO: 153); and CDR3 - QQHYSSPFT (SEQ ID NO: 154), or variants thereof each having up to three amino acid substitutions, additions or deletions; or
o) a heavy chain variable region (VH) having complementarity determining regions (CDRs) with the sequences: CDR1 - IYAMN (SEQ ID NO: 155); CDR2 - RIRSKSNNYARFYADSVKD (SEQ ID NO: 156); and CDR3 - PLRSYFSMDY (SEQ ID NO: 157), or variants thereof each having up to three amino acid substitutions, additions or deletions; and
a light chain variable region (VL) having CDRs with the sequences: CDR1 - KASENVDTYVS (SEQ ID NO: 158); CDR2 - GASNRYT (SEQ ID NO: 159); and CDR3 - GQTYSYPWT (SEQ ID NO: 160), or variants thereof each having up to three amino acid substitutions, additions or deletions.

2. The antigen-binding domain of claim 1, wherein the antigen-binding domain comprises:
a) a VH domain having the sequence of SEQ ID NO: 61; and a VL domain having the sequence of SEQ ID NO: 62 or 108;
b) a VH domain having the sequence of SEQ ID NO: 63; and a VL domain having the sequence of SEQ ID NO: 64 or 109;
c) a VH domain having the sequence of SEQ ID NO: 65; and a VL domain having the sequence of SEQ ID NO: 66;
d) a VH domain having the sequence of SEQ ID NO: 68; and a VL domain having the sequence of SEQ ID NO: 69;
e) a VH domain having the sequence of SEQ ID NO: 71; and a VL domain having the sequence of SEQ ID NO: 72;
f) a VH domain having the sequence of SEQ ID NO: 74; and a VL domain having the sequence of SEQ ID NO: 75;
g) a VH domain having the sequence of SEQ ID NO: 76; and a VL domain having the sequence of SEQ ID NO: 77;
h) a VH domain having the sequence of SEQ ID NO: 78; and a VL domain having the sequence of SEQ ID NO: 79;
i) a VH domain having the sequence of SEQ ID NO: 80; and a VL domain having the sequence of SEQ ID NO: 81; or
j) a VH domain having the sequence of SEQ ID NO: 82; and a VL domain having the sequence of SEQ ID NO: 83;
k) a VH domain having the sequence of SEQ ID NO: 161; and a VL domain having the sequence of SEQ ID NO: 162;
l) a VH domain having the sequence of SEQ ID NO: 163; and a VL domain having the sequence of SEQ ID NO: 164;
m) a VH domain having the sequence of SEQ ID NO: 165; and a VL domain having the sequence of SEQ ID NO: 166;
n) a VH domain having the sequence of SEQ ID NO: 167; and a VL domain having the sequence of SEQ ID NO: 168; or
o) a VH domain having the sequence of SEQ ID NO: 169; and a VL domain having the sequence of SEQ ID NO: 170;
or variants thereof each having at least 90% sequence identity thereto.

3. An antibody comprising the antigen-binding domain of claim 1 or 2.

4. A chimeric antigen receptor (CAR) comprising the antigen-binding domain of claim 1 or 2, optionally wherein
a) the CAR is an scFv CAR;
b) the CAR comprises a CD8a transmembrane domain;
c) the CAR comprises a 4-1BB or a CD28 co-stimulatory domain; and/or
d) the CAR comprises a CD3-zeta signalling domain.

5. A polynucleotide comprising one or more nucleotide sequences encoding the antigen-binding domain of claim 1 or 2, the antibody of claim 3, or the CAR of claim 4.

6. A vector comprising the polynucleotide of claim 5.

7. A cell comprising the polynucleotide of claim 5 or the vector of claim 6.

8. A cell comprising the antigen-binding domain of claim 1 or 2, or the CAR of claim 4.

9. A cell comprising a first CAR and a second CAR, wherein the first CAR is the CAR of claim 4, optionally wherein the second CAR is an anti-CD19 CAR, an anti-CD20 CAR, an anti-CD22 CAR, an anti-CD33 CAR, an anti-CD123 CAR; or an anti-CD7 CAR.

10. The cell of any one of claims 7 to 9, wherein the cell is a T cell or NK cell, optionally wherein the cell is an autologous or allogeneic cell.

11. A pharmaceutical composition comprising the antigen-binding domain of claim 1 or 2, the antibody of claim 3, the CAR of claim 4, the polynucleotide of claim 5, the vector of claim 6, or the cell of any one of claims 7 to 10.

12. The antigen-binding domain of claim 1 or 2, the antibody of claim 3, the CAR of claim 4, the polynucleotide of claim 5, the vector of claim 6, the cell of any one of claims 7 to 10, or the pharmaceutical composition of claim 11 for use in therapy, optionally wherein the therapy is treatment of cancer.

13. The antigen-binding domain, antibody, CAR, polynucleotide, vector, cell, or pharmaceutical composition for use according to claim 12, wherein the cancer is a haematological malignancy, optionally a CD84-expressing haematological malignancy, and/or wherein the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

14. Use of the antigen-binding domain of claim 1 or 2, or the antibody of claim 3, for determining CD84 expression level in a sample, optionally for analysing a CD84 expression level in a sample from a subject.

15. A method for identifying a subject suitable for treatment with an anti-CD84 CAR or antibody, wherein the method comprises determining a CD84 expression level in a sample isolated from the subject, wherein the CD84 expression level is determined using the antigen-binding domain of claim 1 or 2, or the antibody of claim 3.
